# EUROPEAN PATENT SPECIFICATION

(11) **EP 3 978 475 B1**
(45) Date of publication and mention of the grant of the patent: **03.01.2024**
(21) Application number: 20867122.2
(22) Date of filing: 03.08.2020
(51) Int. Cl.: C07D 401/04, C07D 213/06, C07D 213/16, C07D 239/26, C07D 251/24, C07D 401/14, C07D 401/10, H10K 50/00, H10K 85/60

(54) **COMPOUND, PHOTOELECTRIC CONVERSION DEVICE, AND ELECTRONIC DEVICE**
VERBINDUNG, PHOTOELEKTRISCHE UMWANDLUNGSVORRICHTUNG UND ELEKTRONISCHES GERÄT
COMPOSÉ, DISPOSITIF DE CONVERSION PHOTOÉLECTRIQUE ET DISPOSITIF ÉLECTRONIQUE

(30) Priority: 25.09.2019 CN 201910912616
(43) Date of publication of application: 06.04.2022
(73) Proprietor: Shaanxi Lighte Optoelectronics Material Co., Ltd., Xi'an, Shaanxi 710065 (CN)
(72) Inventor: MA, Tiantian, Xi'an Shaanxi 710084 (CN); YANG, Min, Xi'an Shaanxi 710111 (CN); NAN, Peng, Xi'an Shaanxi 710065 (CN)
(74) Representative: Noréns Patentbyrå AB
(86) International application number: PCT/CN2020/106600
(87) International publication number: WO 2021/057264

(56) References cited:
- WO-A1-2020/046049
- WO-A1-2021/057550
- WO-A1-2021/128764
- WO-A1-2021/129072
- WO-A1-2021/135181
- CN-A- 103 503 188
- CN-A- 110 128 279
- CN-A- 110 240 546
- CN-A- 110 240 546
- CN-A- 110 615 759
- CN-A- 111 018 797
- CN-A- 111 039 881
- CN-A- 111 377 853
- CN-A- 111 454 197

## Description

### TECHNICAL FIELD

The present application relates to the technical field of organic materials, in particular to a compound, a photoelectric conversion device and an electronic apparatus.

### BACKGROUND

In recent years, with the development of semiconductor technology, photoelectric conversion devices have been widely used. For example, as a new generation of display device, organic electroluminescent device (OLED) has gradually entered people's vision. Common organic electroluminescent devices are composed of anode, cathode and organic layer between cathode and anode. When a voltage is applied to the cathode and anode, the two electrodes generate an electric field. By the action of electric field, the electrons on the cathode side and the holes on the anode side move to the light emitting layer at the same time, and they combine to form excitons in the light emitting layer. The excitons release energy in the excited state and emit light from the excited state to the ground state.

At present, when organic electroluminescent devices are driven at high temperature, there will be problems such as rising working voltage, reducing luminous efficiency and shortening service life, resulting in the decline of the performance of organic electroluminescent devices.

The existing technical documents have also studied this, such as patent document CN200980125130.5.

WO2021128764 A1 discloses a nitrogen-containing compound, an electronic element, and an electronic device. The structure of the nitrogen-containing compound is as shown in a formula 1 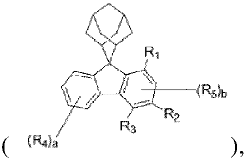 wherein X₁, X₂ and X₃ are respectively and independently selected from C or N, and at least one is N; R₁, R₂ and R₃ are respectively and independently selected from hydrogen or a group as shown in a chemical formula 1-1 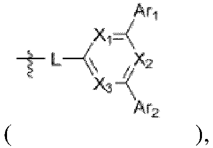 and only one of R₁, R₂ and R₃ is a group as shown in a chemical formula 1-1. The nitrogen-containing compound can improve the performance of the electronic element.

WO2021057550 A1 discloses an organic compound and an electronic device containing the organic compound, which belongs to the technical field of organic materials. The structure of the organic compound is as shown in chemical formula (1) 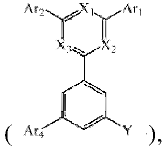 wherein the structure of Y is as shown in formula (2) 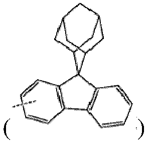 or (3) 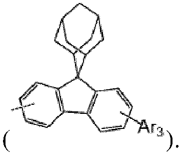 The organic compound is used in an electronic device such as an organic electroluminescent device, and can improve the lifetime property and efficiency property, electrochemical stability and thermal stability and reduce the driving voltage of the organic electroluminescent device.

WO2021135181 A1 discloses a nitrogen-containing compound, an organic electroluminescent device and an electronic device, belonging to the technical field of organic materials. The nitrogen-containing compound of the present application has a carbazole structure and a fluorenyl group, the two cooperating with each other, and has a high first triplet energy level. Therefore, the nitrogen-containing compound of the present application is suitable for use as a host material of a light-emitting layer in an organic electroluminescent device. The nitrogen-containing compound can improve the performance of the organic electroluminescent device.

WO2021129072 A1 discloses an organic compound, an electronic component containing the organic compound, and an electronic device. The compound has a structure as shown by chemical formula 1' 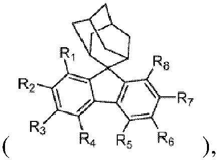 wherein one of R₁ to R₄ is 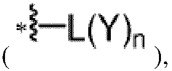 and the other three are selected from substituents such as an alkyl, halogen and cyano; one of R₅ to R₈ is 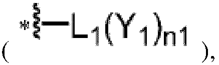 and the other three are selected from substituents such as an alkyl, halogen and cyano; Y and Y₁ are each independently 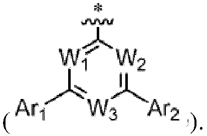 By using the organic compound of the present invention in an electronic component, the driving voltage, luminous efficiency and lifespan of the electronic component can be improved.

WO2020259078 A1 discloses a method for preparing an adamantyl-containing triphenylamine derivative, the method comprising first reacting an aryl halogenated adamantane or an aryl adamantane ester with a primary aromatic amine without separation or purification after a reaction, and directly adding a halogenated aromatic hydrocarbon for reaction. The method not only has fewer reaction steps and can ensure one final separation and purification, but can also reduce the content of impurities, further increases the yield of the product, and prepares the target product in a higher yield. In addition, the steps of the present invention are simple, the raw materials used are basic chemical raw materials, the yield is high, and the finally prepared product has a high purity. The method is superior to the prior art in terms of the cost of raw materials, the actual operability and the working hours required for operation, and is very suitable for industrial production.

CN110128279 A discloses a nitrogen-containing compound, an organic electroluminescent device, and a photoelectric conversion device are provided, which relates to the technical field of electronic components. The nitrogen-containing compound has a structure represented by Chemical Formula 1 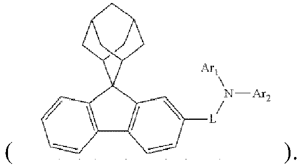 The organic electroluminescent device using the nitrogen-containing compound and the photoelectric conversion device using the nitrogen-containing compound can be improved.

It should be noted that the said information disclosed in the said part of background technology is only used to strengthen the understanding of the background of this application so that it can comprise the information not constituting the existing technologies that are known to the common technicians in this field.

### SUMMARY

The purpose of the present disclosure is to overcome the shortcomings of the prior art and provide a compound and organic electroluminescent device, which can reduce the working voltage, improve the luminous efficiency and prolong the service life of the device.

According to one aspect of the present disclosure, there is provided a compound whose general structural formula is shown in formula 1:

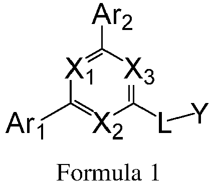

Wherein Y is selected from:

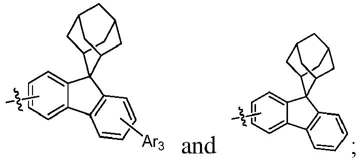

X₁ and X₂ are independently selected from carbon and nitrogen; X₃ is nitrogen;

L is selected from substituted or unsubstituted aryl with 6 to 30 carbon atoms, substituted or unsubstituted heteroaryl with 2 to 29 carbon atoms, substituted or unsubstituted arylalkyl with 7 to 30 carbon atoms, and substituted or unsubstituted heteroarylalkyl with 3 to 29 carbon atoms;

Ar₁, Ar₂ and Ar₃ are the same or different, and are independently selected from substituted or unsubstituted arylalkyl with 7 to 30 carbon atoms, substituted or unsubstituted heteroarylalkyl with 2 to 29 carbon atoms, substituted or unsubstituted aryl with 6 to 30 carbon atoms and substituted or unsubstituted heteroaryl with 2 to 30 carbon atoms;

The substituents of Ar₁, Ar₂, Ar₃ and L are the same or different, and are independently selected from deuterium, cyano, nitro, halogen, hydroxyl, substituted or unsubstituted alkyl with 1 to 24 carbon atoms, substituted or unsubstituted cycloalkyl with 3 to 24 carbon atoms, substituted or unsubstituted alkenyl with 2 to 24 carbon atoms, substituted or unsubstituted alkynyl with 2 to 24 carbon atoms, substituted or unsubstituted heterocycloalkyl with 2 to 24 carbon atoms, substituted or unsubstituted arylalkyl with 7 to 24 carbon atoms, substituted or unsubstituted heteroarylalkyl with 2 to 24 carbon atoms, substituted or unsubstituted aryl with 6 to 24 carbon atoms, substituted or unsubstituted heteroaryl with 1 to 24 carbon atoms, substituted or unsubstituted alkoxy with 1 to 24 carbon atoms, substituted or unsubstituted alkylamino with 1 to 24 carbon atoms, substituted or unsubstituted arylamino with 6 to 24 carbon atoms, substituted or unsubstituted alkylthio with 1 to 24 carbon atoms, substituted or unsubstituted arylalkylamino with 7 to 24 carbon atoms, substituted or unsubstituted heteroarylamino with 1 to 24 carbon atoms, substituted or unsubstituted alkylsilyl with 1 to 24 carbon atoms, substituted or unsubstituted arylsilyl with 6 to 24 carbon atoms, substituted or unsubstituted aryloxy with 6 to 24 carbon atoms and substituted or unsubstituted arylthio with 6 to 24 carbon atoms.

According to one aspect of the present disclosure, a photoelectric conversion device is provided, including an anode and a cathode arranged opposite to each other, and a functional layer arranged between the anode and the cathode;

The functional layer comprises a compound according to any one of the above.

The present disclosure provides compounds, photoelectric conversion devices, and electronic apparatus. In the compound, the core structure of the adamantyl spiro fluorenyl is introduced to the 2,4-diphenyl-1,3,5-triazine which act as the electron injection and transport group. Because the adamantyl spiro fluorenyl has electron rich characteristics, the dipole moment of the whole molecule increases and the polarity is enhanced, which is conducive to the directional arrangement of material molecules, so as to enhance the injection and transmission of electrons and improve the luminous efficiency of the device. At the same time, adamantyl spiro fluorenyl has large molecular weight and steric effect, which can effectively increase the glass transition temperature of the material, inhibit the crystallization of the material, and prolong the service life of the device. In addition, the compound has a LUMO energy level more matched with the adjacent layer, so the prepared organic electroluminescent device has a very low driving voltage. Finally, the adamantyl introduced by the compound increases the molecular weight of the material, reduces the molecular symmetry, improves the evaporation temperature of the material, makes the compound have better physical and thermal stability, good durability and heat resistance, and prolongs the service life of the device.

It should be understood that the above general description and the following detailed description are only exemplary and explanatory and do not limit the present disclosure.

### BRIEF DESCRIPTION OF DRAWINGS

The figures herein are incorporated into and form a part of the description, showing examples consistent with the present disclosure, and are used together with the description to explain the principles of the present disclosure. Obviously, the figures in the following description are only some examples of the present disclosure. For those skilled in the art, other figures can be obtained according to these figures without paying creative labor.
Figure 1 is a structural diagram of an organic electroluminescent device according to an embodiment of the present disclosure.
Figure 2 is a structural diagram of a solar cell according to an embodiment of the present disclosure.

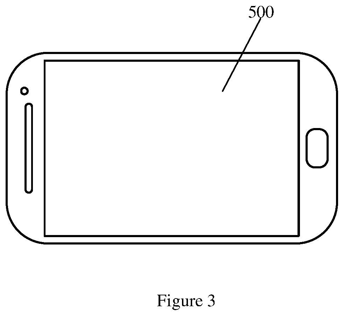

Figure 3 is a schematic diagram of an electronic electronic apparatus according to an embodiment of the present disclosure.

In the figures: 1. Anode; 2. Hole injection layer; 3. Functional layer; 31. Hole transport layer; 32. Electron blocking layer; 33. Electroluminescent layer; 34. Hole blocking layer; 35. Electronic transport layer; 4. Electron injection layer; 5. Cathode; 100 substrate; 200. Anode; 300. Functional layer; 301. Hole transport layer; 302. Photosensitive active layer; 303. Electronic transport layer; 400. Cathode; 500. Screen.

### DETAILED DESCRIPTION OF THE EMBODIMENTS

The sample examples are hereby described more comprehensively by reference to the attached figures. However, the sample examples can be executed in various modes, and should not be understood to be limited to the examples stated here; on the contrary, these examples provided make this disclosure more comprehensive and complete, and pass on the conception of the sample examples to the technicians in this field in a more comprehensive way. The characteristics, structures or properties described can be combined in one or more examples in any proper way. In the description below, many specific details are provided for the full understanding of the examples of this disclosure.

The terms "this" and "described" are used to indicate the existence of one or more elements / components / etc; the terms "include" and "comprise" are used to mean open inclusion and mean that there may be other elements / components / etc. in addition to the listed elements / components / etc.

The embodiment of the present disclosure provides a compound. The general structural formula of the compound is shown in formula 1:

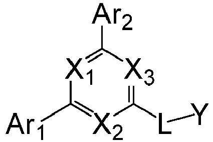

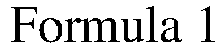

Wherein Y is selected from:

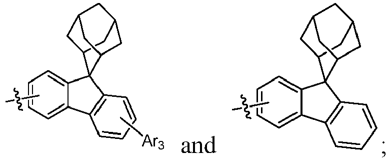

X₁ and X₂ are independently selected from carbon and nitrogen; X₃ is nitrogen;

L is selected from single bond, substituted or unsubstituted aryl with 6 to 30 carbon atoms, substituted or unsubstituted heteroaryl with 2 to 29 carbon atoms, substituted or unsubstituted arylalkyl with 7 to 30 carbon atoms, and substituted or unsubstituted heteroarylalkyl with 3 to 29 carbon atoms;

Ar₁, Ar₂ and Ar₃ are the same or different, and are independently selected from substituted or unsubstituted arylalkyl with 7 to 30 carbon atoms, substituted or unsubstituted heteroarylalkyl with 2 to 29 carbon atoms, substituted or unsubstituted aryl with 6 to 30 carbon atoms, and substituted or unsubstituted heteroaryl with 2 to 30 carbon atoms;

The substituents of Ar₁, Ar₂, Ar₃ and L are the same or different, and are independently selected from deuterium, cyano, nitro, halogen, hydroxyl, substituted or unsubstituted alkyl with 1 to 24 carbon atoms, substituted or unsubstituted cycloalkyl with 3 to 24 carbon atoms, substituted or unsubstituted alkenyl with 2 to 24 carbon atoms, substituted or unsubstituted alkynyl with 2 to 24 carbon atoms, substituted or unsubstituted heterocycloalkyl with 2 to 24 carbon atoms, substituted or unsubstituted arylalkyl with 7 to 24 carbon atoms, substituted or unsubstituted heteroarylalkyl with 2 to 24 carbon atoms, substituted or unsubstituted aryl with 6 to 24 carbon atoms, substituted or unsubstituted heteroaryl with 1 to 24 carbon atoms, substituted or unsubstituted alkoxy with 1 to 24 carbon atoms, substituted or unsubstituted alkylamino with 1 to 24 carbon atoms, substituted or unsubstituted arylamino with 6 to 24 carbon atoms, substituted or unsubstituted alkylthio with 1 to 24 carbon atoms, substituted or unsubstituted arylalkylamino with 7 to 24 carbon atoms, substituted or unsubstituted heteroarylamino with 1 to 24 carbon atoms, substituted or unsubstituted alkylsilyl with 1 to 24 carbon atoms, substituted or unsubstituted arylsilyl with 6 to 24 carbon atoms, substituted or unsubstituted aryloxy with 6 to 24 carbon atoms, and substituted or unsubstituted arylthio with 6 to 24 carbon atoms.

The present disclosure provides compounds, photoelectric conversion devices, and electronic apparatus. In the compound, the core structure of the adamantyl spiro fluorenyl is introduced to the 2,4-diphenyl-1,3,5-triazine which act as the electron injection and transport group. Because the adamantyl spiro fluorenyl has electron rich characteristics, the polarity of the whole molecule is enhanced, which is conducive to the directional arrangement of material molecules, so as to enhance the injection and transmission of electrons and improve the luminous efficiency of the device. At the same time, adamantyl spiro fluorenyl has large molecular weight and steric effect, which can effectively increase the glass transition temperature of the material, inhibit the crystallization of the material, and prolong the service life of the device. In addition, the compound has a LUMO energy level more matched with the adjacent layer, so the prepared organic electroluminescent device has a very low driving voltage. Finally, the adamantyl introduced by the compound increases the molecular weight of the material, reduces the molecular symmetry, improves the evaporation temperature of the material, makes the compound have better physical and thermal stability, good durability and heat resistance, and prolongs the service life of the device.

Each part of the compound according to the example of the present disclosure is described in detail below:

The general structural formula of the compound of the present disclosure can be shown in formula 1:

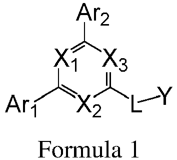

Wherein Y is selected from:

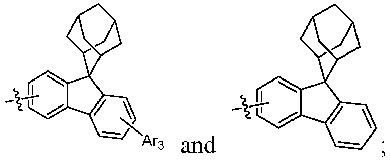

Wherein X₁ and X₂ are independently selected from carbon and nitrogen; X₃ is nitrogen;.

In the present disclosure, the number of carbon atoms of Ar₁, Ar₂, Ar₃ and L refers to the number of all carbon atoms. For example, if Ar₁ is selected from the substituted arylene with 10 carbon atoms, the number of all carbon atoms of the arylene and its substituents is 10; if Ar₁ is 9,9-dimethylfluorenyl, the substituted fluorenyl has 15 carbon atoms and the ring-forming carbon atom number of Ar₁ is 13.

In the present disclosure, "substituted or unsubstituted" means that there is no substituent or is substituted by one or more substituents. The substituents include, but are not limited to, deuterium, halogen (F, Cl, Br), cyano, alkyl, alkoxy, haloalkyl, alkenyl, alkynyl, haloalkyl, aryl, heteroaryl, aryloxy, arylthio, silyl, alkylamino, cycloalkyl, heterocyclic, alkylsilyl, arylsilyl.

In the present disclosure, "optional" or "optionally" means that the event or environment described later can but need not occur. The description includes the occasions where the event or environment occurs or does not occur. "Optionally substituted by..." means that the group can be substituted by the substituent or unsubstituted by the substituent.

In the present disclosure, 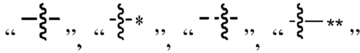 , "- - -" have the same meaning, and all represent single bonds interconnected between groups.

In the present disclosure, "alkyl" may include linear alkyl or branched alkyl. Alkyl may have 1 to 24 carbon atoms, and in the present disclosure, a numerical range such as "1 to 24" refers to each integer in the given range. The alkyl may be a lower alkyl having 1 to 6 carbon atoms. In some embodiments, the alkyl contains 1 to 4 carbon atoms; in other embodiments, the alkyl contains 1 to 3 carbon atoms. The alkyl may optionally be substituted by one or more substituents described in the present invention. Examples of alkyl include, but are not limited to, methyl (Me, -CH₃), ethyl (Et, -CH₂CH₃), n-propyl (n-Pr, -CH₂CH₂CH₃), isopropyl (i-Pr, - CH(CH₃)₂), n-butyl (n-Bu, -CH₂CH₂CH₂CH₃), isobutyl (i-Bu, -CH₂CH(CH₃)₂), SEC butyl (s-Bu, -CH(CH₃)CH₂CH₃), tert butyl (t-Bu, -C(CH₃)₃), etc. In addition, alkyl may be substituted or unsubstituted.

In this disclosure, halogen as a substituent refers to fluorine, chlorine, bromine or iodine.

In the present disclosure, aryl refers to an optional functional group or substituent derived from the aromatic hydrocarbon ring. The aryl may be a monocyclic aryl or a polycyclic aryl, in other words, the aryl may be a monocyclic aryl, a fused aryl, two or more monocyclic aryl conjugated by a carbon-carbon bond, a monocyclic aryl and a fused aryl conjugated by a carbon-carbon bond, and two or more fused aryl conjugated by a carbon-carbon bond. That is, two or more aromatic groups conjugated by carbon-carbon bonds can also be regarded as aryl of the present disclosure. Fused aryl refers to two or more rings in which two carbon atoms in the ring system are shared by two adjacent rings, wherein at least one of the rings is aromatic, for example, other rings can be cycloalkyl, cycloalkenyl and aryl. For example, in the present disclosure, biphenyl, terphenyl, etc. are aryl. Examples of aryl may include, but are not limited to, phenyl, naphthyl, fluorenyl, anthracyl, phenanthrene, biphenyl, terphenyl, tetradiphenyl, pentadiphenyl, hexadiphenyl, benzo [9,10] phenanthryl, pyrenyl, perylenyl, benzofluoranthenyl, chrysene base, 9,9 dimethylfluorenyl, 9,9-diphenylfluorenyl, spirodifluorenyl, indenyl, etc.

In the present disclosure, the substituted aryl may be one or more hydrogen atoms in the aryl, which are replaced by groups such as deuterium atom, halogen, cyano, aryl, heteroaryl, alkylsilyl, arylsilyl, alkyl, haloalkyl, cycloalkyl, alkoxy, alkylthio, etc. Specific examples of heteroaryl substituted aryl include, but are not limited to, dibenzofuranyl-substituted phenyl, dibenzothiophenyl-substituted phenyl, pyridinyl-substituted phenyl, carbazolyl-substituted phenyl, etc; specific examples of aryl substituted aryl include, but are not limited to, naphthyl substituted phenyl, phenyl substituted naphthyl, phenanthrenyl-substituted phenyl, phenyl-substituted anthracenyl, anthracenyl-substituted phenyl, etc. It should be understood that the number of carbon atoms of substituted aryl refers to the total number of carbon atoms of aryl and substituents on aryl. For example, a substituted aryl with 18 carbon atoms means that the total number of carbon atoms of aryl and substituent groups is 18.

In the present disclosure, the heteroaryl may be a heteroaryl including at least one of B, O, N, P, Si, Se and S as a heteroatom. The heteroaryl can be a monocyclic heteroaryl or a polycyclic heteroaryl. In other words, the heteroaryl can be a single aromatic ring system or a plurality of aromatic ring systems conjugated by carbon-carbon bonds. Any aromatic ring system is an aromatic monocyclic ring or an aromatic fused ring, and any aromatic ring system contains the heteroatom. Fused heteroaryl refers to two or more rings in which two atoms in the ring system are shared by two adjacent rings, wherein at least one of the rings is aromatic, for example, other rings can be cycloalkyl, heterocyclic, cycloalkenyl and aryl.

For example, heteroaryl may include thiophenyl, furanyl, pyrrolyl, imidazolyl, thiazolyl, oxazolyl, oxadiazolyl, triazolyl, pyridyl, bipyridyl, pyrimidinyl, triazinyl, acridinyl, pyridazinyl, pyrazinyl, quinolinyl, quinazolinyl, quinoxalinyl, phenoxazinyl, pyridopyrimidinyl, pyridopyrazinyl, pyrazinopyrazinyl, isoquinolinyl, indolyl, carbazolyl, benzoxazolyl, benzimidazolyl, benzothiazolyl, benzocarbazolyl, benzothiophenyl, dibenzothiophenyl, thiophenothiophenyl, benzofuranyl, phenanthrolinyl, isoxazolyl, thiadiazolyl, benzothiazolyl, phenothiazinyl, silicofluorenyl, dibenzofuranyl and N-arylcarbazolyl (e.g. N-phenylcarbazolyl), N-heteroarylcarbazolyl (e.g. A-pyridyl-carbazolyl), N-alkyl carbazolyl (e.g. N-methylcarbazole), etc., but are not limited to these.

In the present disclosure, the substituted heteroaryl may be that one or more hydrogen atoms in the heteroaryl are replaced by groups such as deuterium atom, halogen, cyano, aryl, heteroaryl, trialkylsilyl, alkyl, cycloalkyl, alkoxy, alkylthio, etc. Specific examples of aryl substituted heteroaryl include, but are not limited to, phenyl substituted dibenzofuryl, phenyl substituted dibenzothiophenyl, phenyl substituted pyridyl, phenyl substituted carbazolyl, etc. It should be understood that the number of carbon atoms of substituted heteroaryl refers to the total number of carbon atoms of heteroaryl and substituents groups on heteroaryl. For example, a substituted heteroaryl with 14 carbon atoms means that the total number of carbon atoms of heteroaryl and substituent group is 14.

In the present disclosure, the so-called number of ring-forming atoms refers to the number of atoms constituting the ring itself in the structure formed by the synthesis of a ring by atomic bond (such as monocyclic compound, fused ring compound, carbocyclic compound and heterocyclic compound). The number of ring-forming atoms does not include atoms that do not form a ring (such as hydrogen atoms that end the chemical bond of the atoms that form a ring), and atoms contained in the substituent when the ring is replaced by a substituent. "Number of ring-forming atoms" means the same unless otherwise specified. For example, the number of ring-forming atoms of pyridine ring is 6, the number of ring-forming atoms of quinazoline ring is 10, and the number of ring-forming atoms of furan ring is 5. The number of ring-forming atoms does not include the hydrogen atoms bonded on the carbon atoms of pyridine ring and quinazoline ring respectively and the atoms constituting substituents. In addition, when phenyl as a substituent is connected to the fluorene ring, the number of ring-forming atoms does not include the number of atoms in phenyl as a substituent. When a cyclic group such as a fluorene ring (including a spiro fluorene ring) is spirocheted on the fluorene, the number of ring atoms includes the number of spirofluorene rings atoms.

In some embodiments, in the compound shown in formula 1, L may be selected from single bond, substituted or unsubstituted aryl with 6 to 30 carbon atoms, substituted or unsubstituted heteroaryl with 2 to 29 carbon atoms, substituted or unsubstituted arylalkyl with 7 to 30 carbon atoms, and substituted or unsubstituted heteroarylalkyl with 3 to 29 carbon atoms.

In some embodiments, in the compound shown in formula 1, L is selected from the following substituted or unsubstituted groups: phenylene, phenylene substituted by benzene, biphenylene, terphenylene, naphthylene, dibenzofurylene, dibenzothienylene, diphenylfluorenylidene, spirobifluorenylidene, anthrylene, phenanthrylene, carbazolylidene, N-phenylcarbazolylene. The substitution means that it is optionally substituted by a substituent selected from deuterium, fluorine, cyano, methyl, ethyl, isopropyl, tert butyl, phenyl, pyridyl and carbazole.

In some embodiments, in the compound shown in formula 1, L may be selected from arylene with 6 to 25 ring-forming carbon atoms.

For example, L may be selected from the group formed by the following groups:

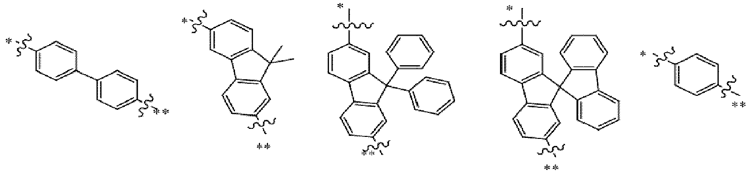

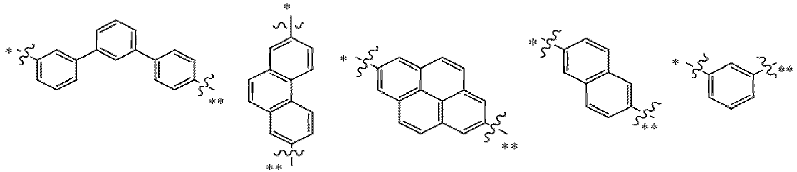

Wherein * can represent that the above group is used for binding with the group of 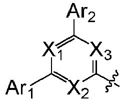 in formula 1;

**It can be expressed that the above groups are used for binding with the group of 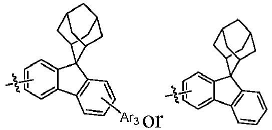 in formula 1.

In some embodiments, among the compounds shown in formula 1, Ar₁, Ar₂ and Ar₃ are the same or different, and are independently selected from substituted or unsubstituted arylalkyl with 7 to 30 carbon atoms, substituted or unsubstituted heteroarylalkyl with 2 to 29 carbon atoms, substituted or unsubstituted aryl with 6 to 30 carbon atoms, and substituted or unsubstituted heteroaryl with 2 to 30 carbon atoms;

The substituents of Ar₁, Ar₂, Ar₃ and L are the same or different, and are independently selected from deuterium, cyano, nitro, halogen, hydroxyl, substituted or unsubstituted alkyl with 1 to 24 carbon atoms, substituted or unsubstituted cycloalkyl with 3 to 24 carbon atoms, substituted or unsubstituted alkenyl with 2 to 24 carbon atoms, substituted or unsubstituted alkynyl with 2 to 24 carbon atoms, substituted or unsubstituted heterocycloalkyl with 2 to 24 carbon atoms, substituted or unsubstituted arylalkyl with 7 to 24 carbon atoms, substituted or unsubstituted heteroarylalkyl with 2 to 24 carbon atoms, substituted or unsubstituted aryl with 6 to 24 carbon atoms, substituted or unsubstituted heteroaryl with 1 to 24 carbon atoms, substituted or unsubstituted alkoxy with 1 to 24 carbon atoms, substituted or unsubstituted alkylamino with 1 to 24 carbon atoms, substituted or unsubstituted arylamino with 6 to 24 carbon atoms, substituted or unsubstituted alkylthio with 1 to 24 carbon atoms, substituted or unsubstituted arylalkylamino with 7 to 24 carbon atoms, substituted or unsubstituted heteroarylamino with 1 to 24 carbon atoms, substituted or unsubstituted alkylsilyl with 1-24 carbon atoms, substituted or unsubstituted arylsilyl with 6 to 24 carbon atoms, substituted or unsubstituted aryloxy with 6 to 24 carbon atoms, and substituted or unsubstituted arylthio with 6 to 24 carbon atoms.

Wherein "hetero" refers to that one functional group includes 1 to 3 heteroatoms selected from the group composed of B, N, O, S and P, and the rest is carbon.

In another embodiments, Ar₁ and Ar₂ are the same or different, and are independently selected from substituted or unsubstituted aryl with 6 to 30 carbon atoms and substituted or unsubstituted heteroaryl with 2 to 29 carbon atoms.

For example, Ar₁ may be selected from substituted or unsubstituted aryl with 6 to 30 carbon atoms; Ar₂ can be selected from substituted or unsubstituted heteroaryl with 2 to 29 carbon atoms. Of course, Ar₁ and Ar₂ can also be selected from substituted or unsubstituted aryl with 6 to 30 carbon atoms, and substituted or unsubstituted heteroaryl with 2 to 29 carbon atoms.

In another embodiments, Ar₁ and Ar₂ are the same or different, and are independently selected from substituted or unsubstituted aryl with 6 to 12 ring-forming carbon atoms and substituted or unsubstituted heteroaryl with 5 ring-forming carbon atoms.

For example, Ar₁ may be selected from: substituted or unsubstituted aryl with 6 to 12 ring-forming carbon atoms; Ar₂ may be selected from substituted or unsubstituted heteroaryl with 5 ring-forming carbon atoms. Of course, Ar₁ and Ar₂ can also be selected from substituted or unsubstituted aryl with 6 to 12 ring-forming carbon atoms, and substituted or unsubstituted heteroaryl with 5 ring-forming carbon atoms.

In some embodiments, Ar₁ and Ar₂ are the same or different, and are independently selected from the following substituted or unsubstituted groups: phenyl, biphenyl, terphenyl, naphthyl, anthracyl, phenanthryl, dibenzofuranyl, dibenzothiophenyl, carbazolyl, N-phenylcarbazolyl, fluorenyl, spirodifluorenyl, phenanthrolinyl and pyridyl, respectively. The substitution refers to being optionally substituted by a substituent selected from deuterium, fluorine, cyano, methyl, ethyl, isopropyl, tert-butyl, phenyl, naphthyl, biphenyl, pyridyl, dibenzofuranyl, dibenzothiophenyl and carbazole.

In some more specific embodiments, Ar₁ and Ar₂ are the same or different and are independently selected from the group formed by the following groups:

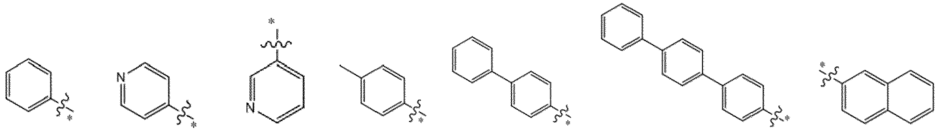

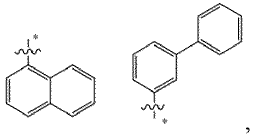

Wherein * can represent that the above group is used for binding with the 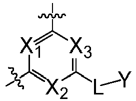 group in formula 1.

For example, the compound shown in formula 1 may be selected from the group formed by the following compounds:

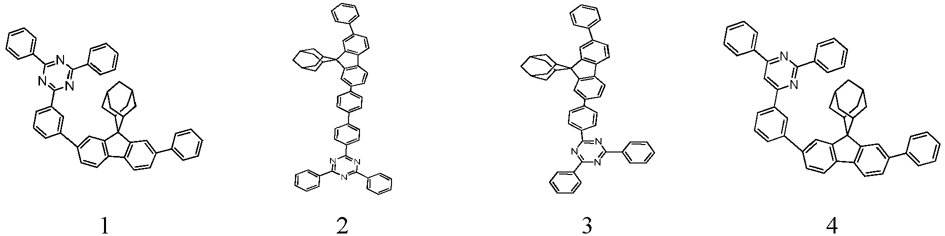

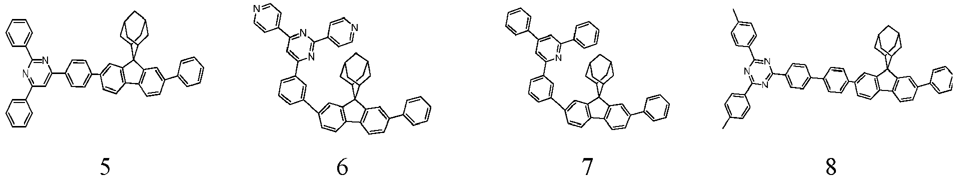

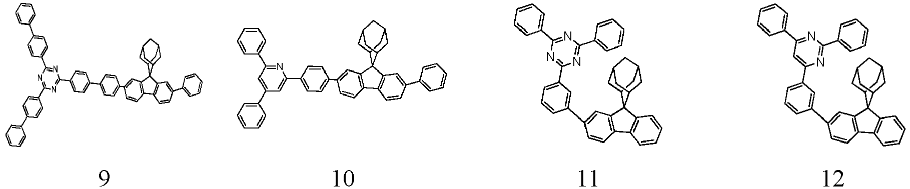

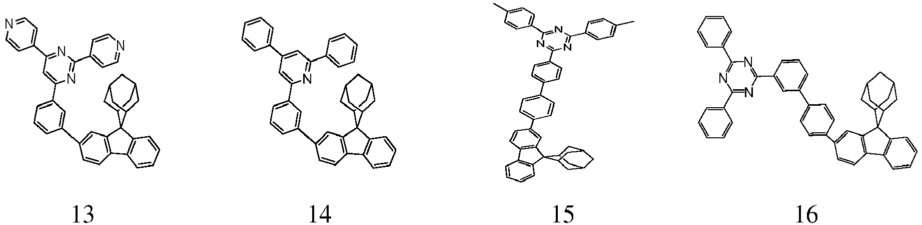

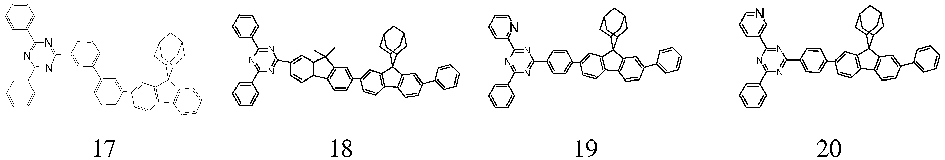

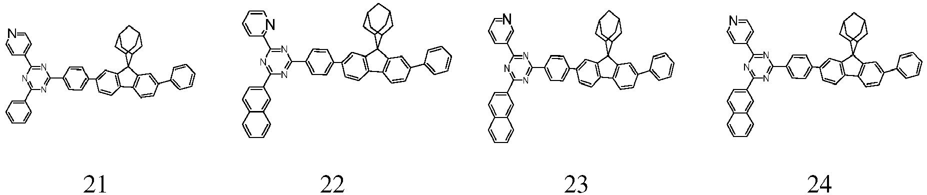

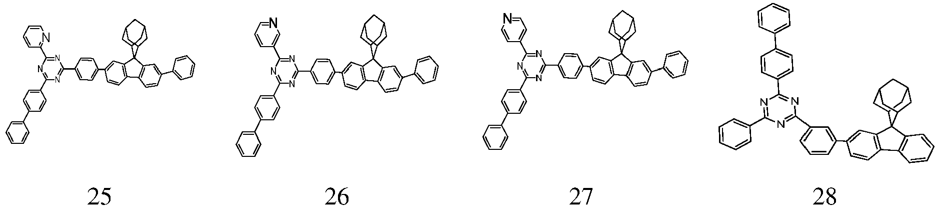

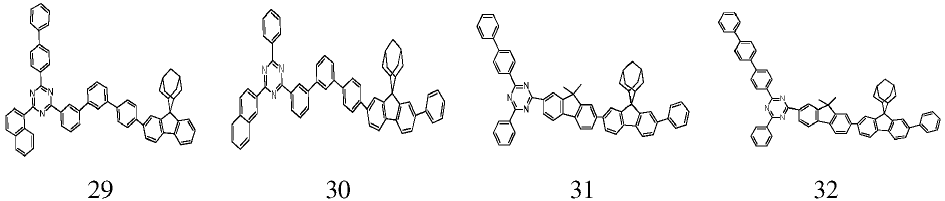

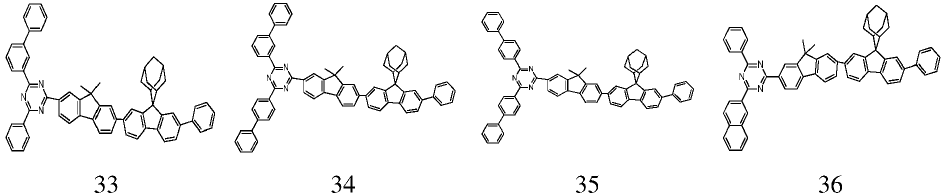

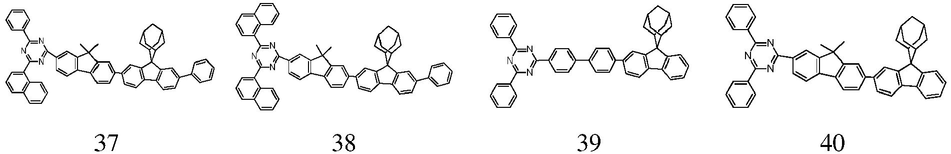

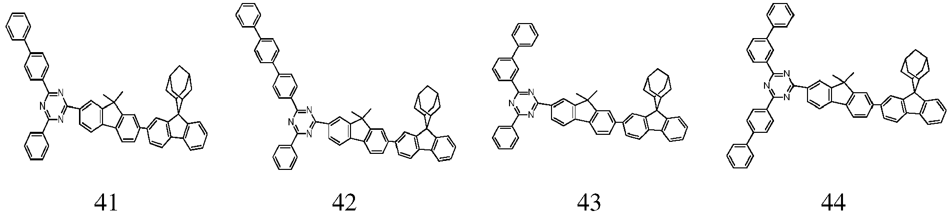

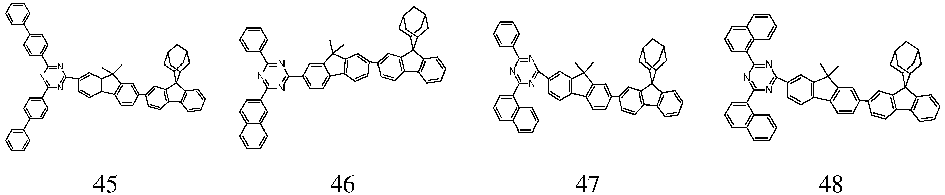

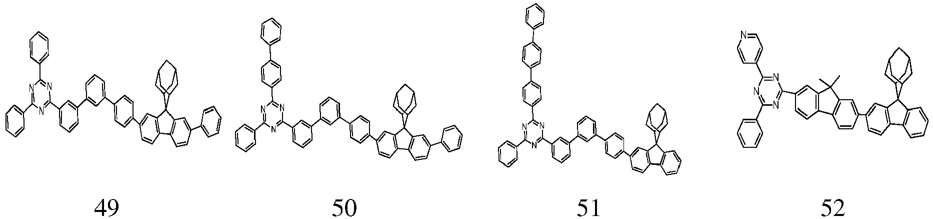

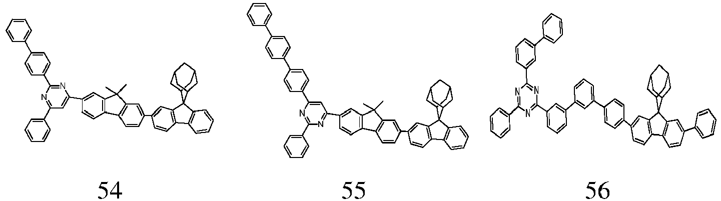

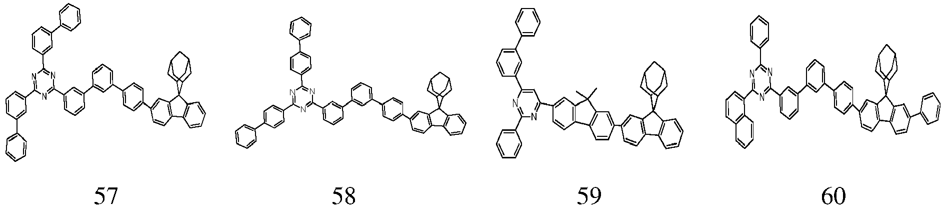

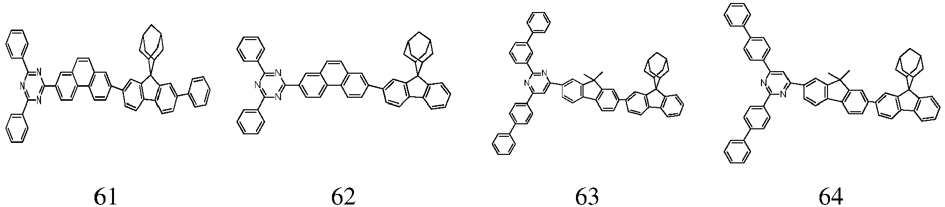

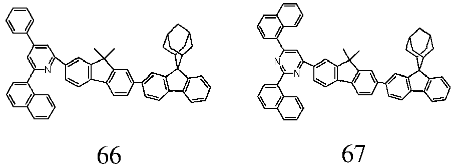

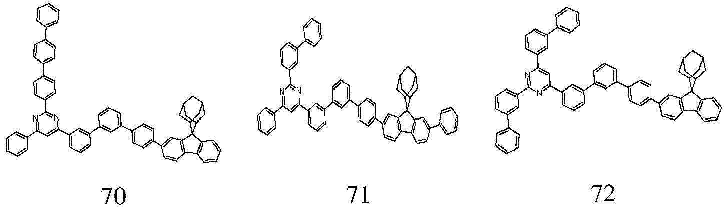

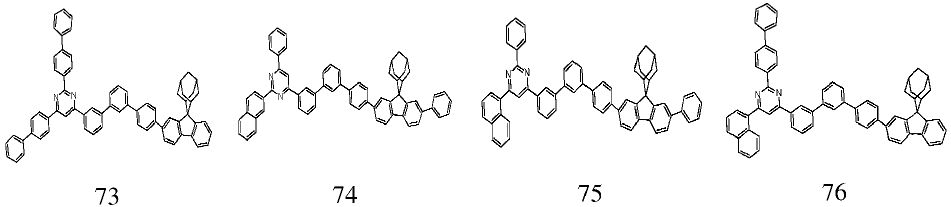

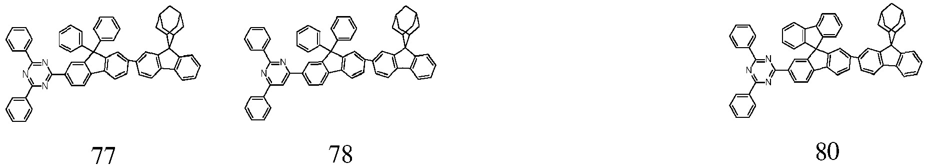

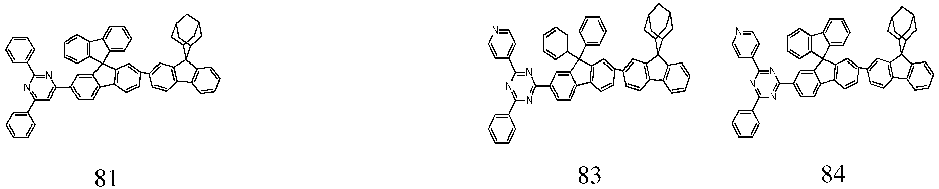

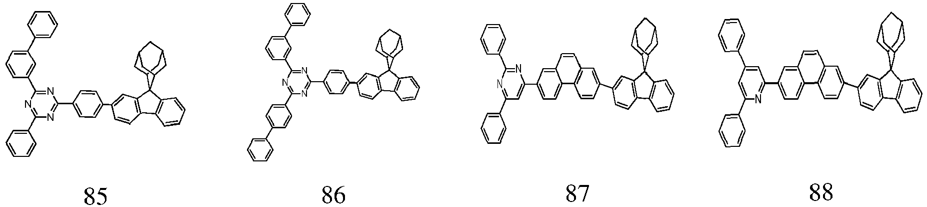

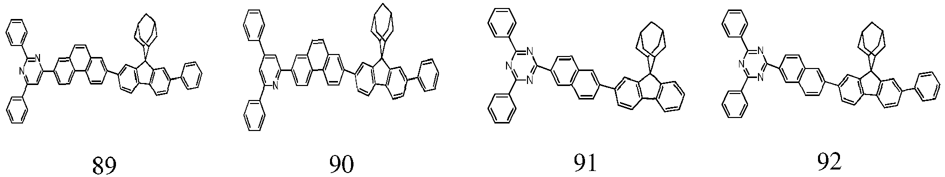

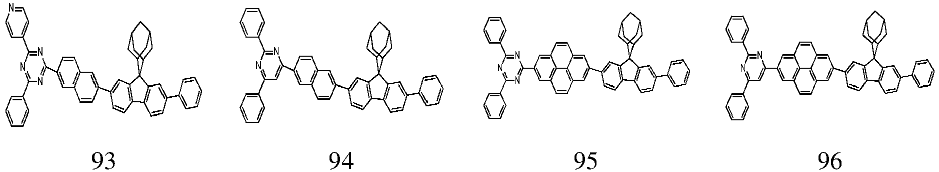

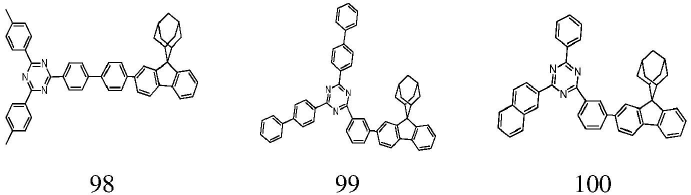

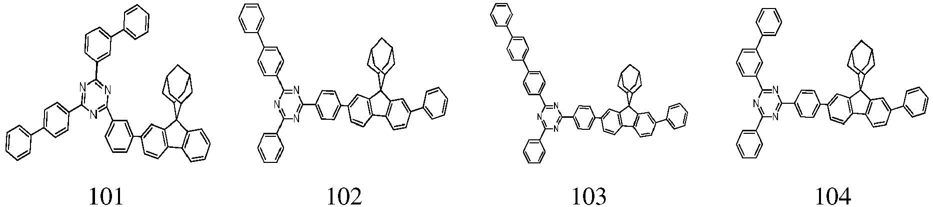

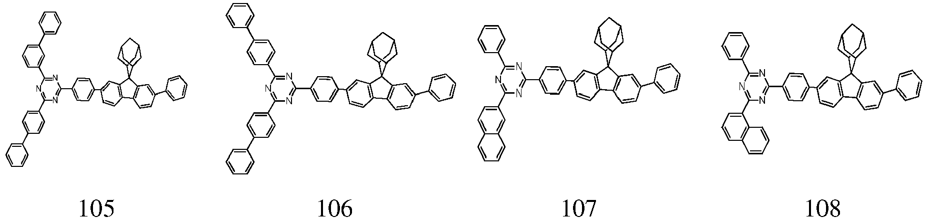

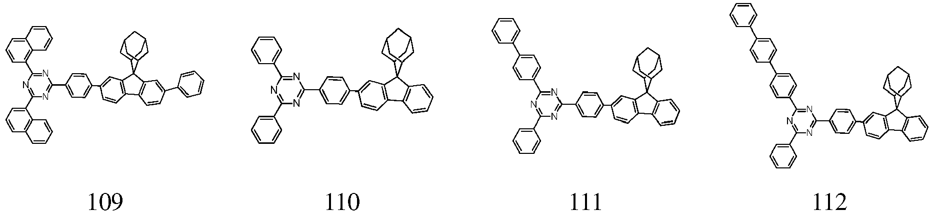

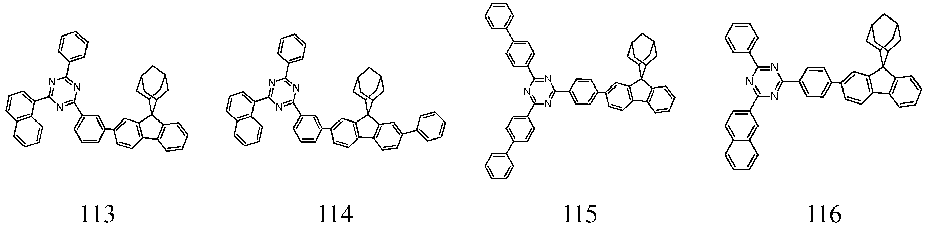

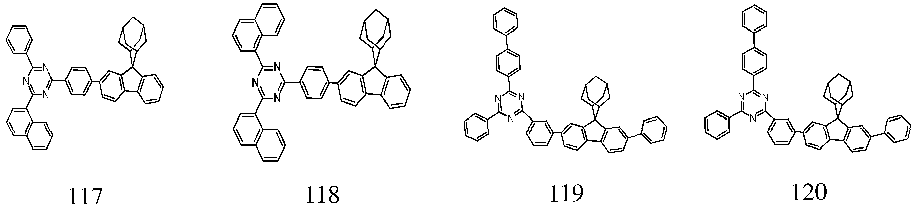

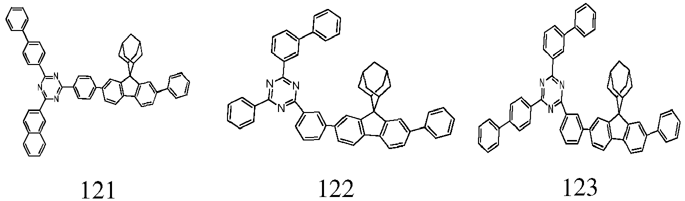

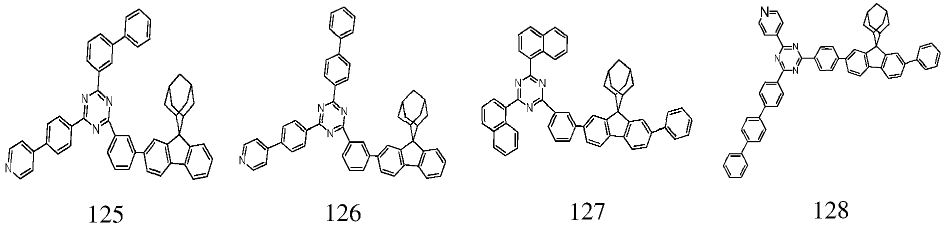

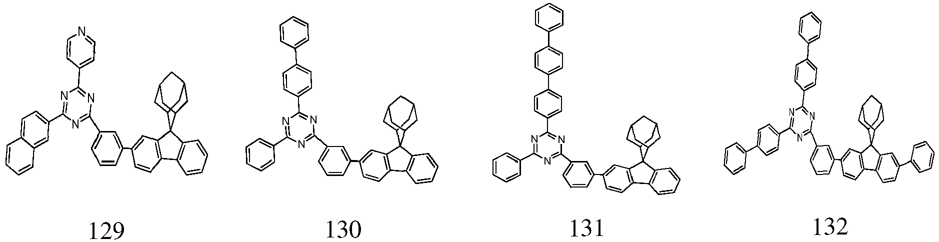

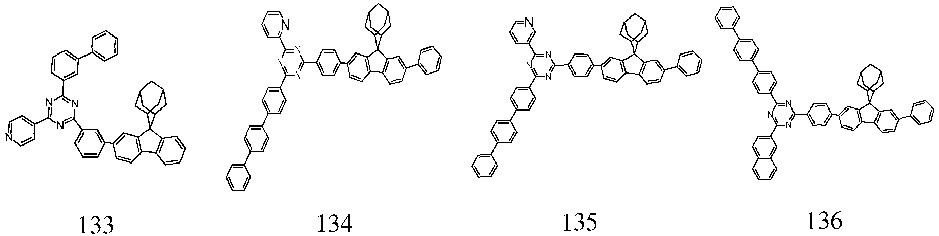

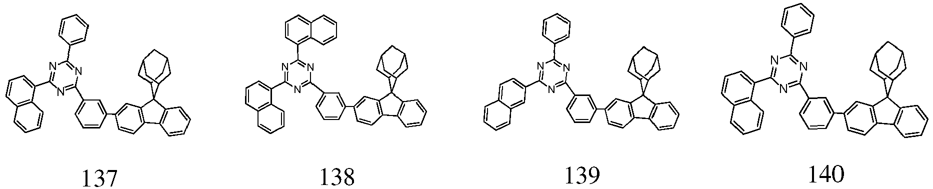

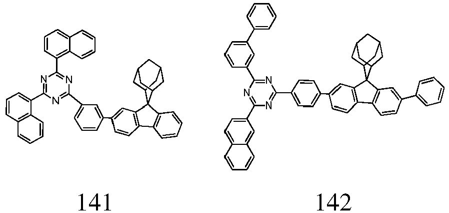

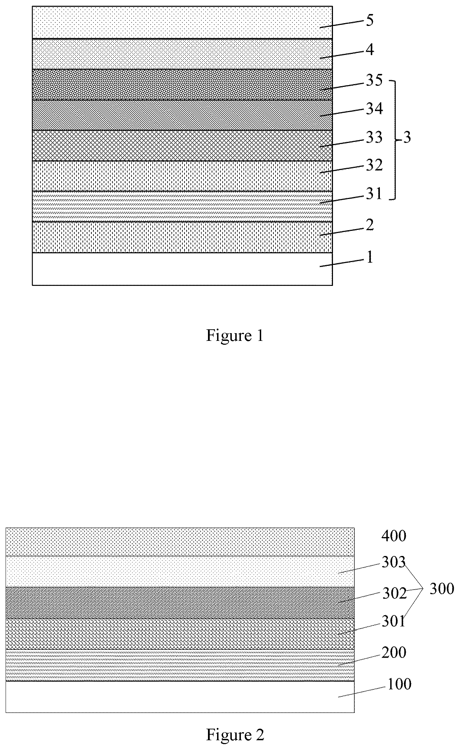

The present disclosure also provides a photoelectric conversion device. As shown in Figure 1, the photoelectric conversion device may include an anode 1 and a cathode 5 arranged relatively, and a functional layer 3 arranged between the anode 1 and the cathode 5. The functional layer 3 may include a compound provided by any of the above examples.

The anode 1 may be a material that facilitates hole injection into the functional layer 3, which may have a large work function. For example, the anode 1 material may be a metal, an alloy or a metal oxide, for example, it may be nickel, platinum, vanadium, chromium, copper, zinc, gold or their alloys, or it may be zinc oxide, indium oxide, indium tin oxide (ITO) and indium zinc oxide (IZO); Of course, the anode 1 material can also be other, for example, it can also be a composition, such as ZnO: Al, SnO₂: Sb, conductive polymer (poly (3-methylthiophene), poly [3,4 - (ethylidene-1,2-dioxyl) thiophene] (PEDT), polypyrrole and polyaniline. Of course, anode 1 materials are not limited to this, but can also be other materials, which will not be listed one by one here. Preferably, the anode 1 material may be indium tin oxide (ITO).

The cathode 5 may be a material that facilitates electron injection into the functional layer 3, which may have a small work function. For example, the cathode 5 material can be a metal or alloy material, for example, it can be magnesium, calcium, sodium, potassium, titanium, indium, yttrium, lithium, gadolinium, aluminum, silver, tin, lead or their alloys, or multi-layer materials, such as LiF/Al, Liq/Al, LiO₂/Al, LiF/Ca, LiF/Al and BaF₂/Ca. Of course, cathode 5 materials are not limited to this, but can also be other materials, which is not be listed one by one here. Preferably, the cathode 5 material may be aluminum. For example, the photoelectric conversion device can be an organic electroluminescent device, and of course, it can also be other photoelectric conversion devices, which is not be listed one by one here.

The compound according to any example of the present disclosure can be used to form one or more layers in the functional layer 3 to reduce the working voltage of the photoelectric conversion device, improve the luminous efficiency and prolong the service life of the device. Specifically, the functional layer 3 may include an electron transport layer 35, which may include a compound according to any example of the present disclosure. The compound can also be used as a light-emitting host. For example, the compound can be used as a phosphorescent or fluorescent host material, a blue light-emitting dopant material or an electron transport material.

The functional layer 3 may also include a light emitting layer 33 and a hole transport layer 31, wherein the light emitting layer 33 may be arranged on the side of the electron transport layer 35 away from the cathode 5; the hole transport layer 31 may be arranged on the side of the light emitting layer 33 away from the cathode 5. The photoelectric conversion device may include a stacked anode 1, a hole transport layer 31, a light emitting layer 33, an electron transport layer 35 and a cathode 5. Hole transport materials can be selected from carbazole polymers, carbazole linked triarylamines or other types of compounds, which are not specially limited in the present disclosure. For example, in one embodiment of the present disclosure, the hole transport layer 31 is composed of a compound TPD or NPB. The main material of the organic light emitting layer 33 may be a metal chelate compound, a diphenylvinyl derivative, an aromatic amine derivative, a dibenzofuran derivative or other types of materials, which is not particularly limited in the present disclosure. In one embodiment of the present disclosure, the main material of the organic light emitting layer 33 may be CBP or α, β-AND, the dopant is 4,4'- (3,8-diphenylpyrene-1,6-diyl) bis (N,N-diphenylaniline).

The compound provided by the present disclosure can be used in the electron transport layer 35 of the photoelectric conversion device and can be used to improve the electronic characteristics of the photoelectric conversion device, wherein the electronic characteristics can mean that the electrons formed in the cathode 5 can be easily injected into the light emitting layer and can be transmitted in the light emitting layer according to the conduction characteristics of the LUMO level.

Meanwhile, the photoelectric conversion device according to the embodiment of the present disclosure may also include a hole injection layer 2, an electron injection layer 4, an electron blocking layer 32 or a hole blocking layer 34, wherein the hole injection layer 2 may be arranged between the anode 1 and the hole transport layer 31; the electron injection layer 4 may be arranged between the cathode 5 and the electron transport layer 35; the electron blocking layer 32 may be arranged between the light emitting layer 33 and the hole transport layer 31; the hole blocking layer 34 may be arranged between the light emitting layer 33 and the electron transport layer 35.

The hole injection layer 2 can select benzidine derivatives, starburst arylamine compounds, phthalocyanine derivatives or other materials, which are not specially limited in the present disclosure. In one embodiment of the present disclosure, the hole injection layer 2 may be composed of HAT-CN or m-MTDATA. The electron injection layer 4 may include inorganic materials such as alkali metal sulfide and alkali metal halide, or may include a complex of alkali metal and organic matter. In one embodiment of the present disclosure, the electron injection layer 4 may include ytterbium (Yb). The electron barrier material can be selected from carbazole polymers or other types of compounds, which is not specially limited in the present disclosure. For example, in some embodiments of the present disclosure, the electron blocking layer 32 consists of a compound TCTA.

In other embodiments, the photoelectric conversion device may also be a solar cell, as shown in Figure 2, for example, it may be an organic solar cell. It mainly includes a cathode 400, an anode 200 and a functional layer 300. The functional layer 300 may be arranged between the cathode 400 and the anode 200, and the functional layer 300 may include a nitrogen-containing compound in any embodiment of the present disclosure, which can be used to improve the exciton transmission rate. In one embodiment, the functional layer 300 may include an electron transport layer 303, a hole transport layer 301 and a photosensitive active layer 302, an anode 200 may be formed on a substrate 100, the anode 200 may be a film attached to the substrate 100, a hole transport layer 301 may be formed on the surface of the anode 200 away from the substrate 100, and a photosensitive active layer 302 may be formed on the surface of the hole transport layer 301 away from the anode 200. An electron transport layer 303 may be formed on the surface of the photosensitive active layer 302 away from the hole transport layer 301, and the electron transport layer 303 may include a nitrogen-containing compound according to any embodiment of the present disclosure, and a cathode 400 may be formed on the surface of the electron transport layer 303 away from the photosensitive active layer 302. When sunlight irradiates the solar cell, the electrons in the photosensitive active layer 302 obtain energy and transition to produce excitons. With the assistance of the electron transport layer 303 and the hole transport layer 301, the electrons move to the cathode 400 and the holes move to the anode 200, so that a potential difference can be generated between the cathode 400 and the anode 200 of the solar cell, so as to realize the power generation function. In this process, the compound of the present disclosure can be used to enhance the transmission rate of electrons in the electron transport layer 303, avoid recombination of electrons and holes, and then increase the number of electrons transmitted to the cathode 400, so as to improve the open circuit voltage of the solar cell and improve the photoelectric conversion efficiency. The disclosure also provides an electronic apparatus, which can include a photoelectric conversion device according to any of the above embodiments. The beneficial effects and specific details can refer to the above photoelectric conversion devices, which will not be repeated here. For example, the electronic apparatus can be a display, array substrate, photovoltaic module, etc. Of course, it can also be other devices, which will not be listed one by one here. For example, it can be the screen 500 of a mobile phone, camera or computer, and of course, it can also be other devices, which are not specially limited here.

Next, the compounds and photoelectric conversion devices of the present disclosure will be described in detail through examples. However, the following examples are only examples of the present disclosure and do not limit the present disclosure.

Generally, the organic compound of the disclosure can be prepared by the method described in the disclosure. Those skilled in the art will recognize that the chemical reactions described in this disclosure can be used to appropriately prepare many other compounds of this disclosure, and other methods for preparing organic compounds of this disclosure are considered to be within the scope of this disclosure. For example, those skilled in the art can synthesize other organic compounds of the disclosure by referring to or appropriately modifying the preparation method provided by the disclosure, for example, they can use appropriate protective groups, use other known reagents other than those described in the disclosure, and modify reaction conditions, etc.

In the synthesis example described below, the temperature is Celsius unless otherwise stated.

Some reagents are purchased from commodity suppliers, such as Aldrich Chemical Company, Arco Chemical Company and Alfa Chemical Company. Unless otherwise stated, these reagents are used without further purification. Some conventional reagents were purchased from Shantou Xilong Chemical Factory, Guangdong Guanghua Chemical Reagent Factory, Guangzhou Chemical Reagent Factory, Tianjin Haoyuyu Chemical Co., Ltd., Tianjin Fuchen Chemical Reagent Factory, Qingdao Tenglong Chemical Reagent Co., Ltd. and Qingdao Marine Chemical Factory. The raw materials used are commercially purchased.

Unless otherwise stated, the determination condition of low resolution mass spectrometry (MS) data is Agilent 6120 four-stage rod HPLC-M (column model: Zorbax SB-C18, 2.1×30mm, 3.5µm, 6min, flow rate 0.6ml/min. Mobile phase: 5%-95% (the ratio of acetonitrile containing 0.1% formic acid to H2O (containing 0.1% formic acid)), detected by electrospray ionization(ESI) and UV at 210nm/254nm.

1H NMR spectra are recorded by Bruker 400MHz or 600MHz NMR spectrometer. 1H NMR spectra take CDCl₃, CD₂Cl₂, D₂O, DMSO-*d₆*, CD₃OD or acetone-*d₆* as solvents (in ppm), and TMS (0ppm) or chloroform (7.26ppm) as reference standards. When multiple peaks appear, the following abbreviations will be used: s (singlet), d (doublet), t (triplet), m (multiplet), br (broadened), dd (double of doublets)

Pure compounds are detected with UV at 210nm / 254nm by using Agilent 1260pre-HPLC or Calesep pump 250pre-HPLC (column model: NOVASEP 50/80mm DAC).

### Synthesis Example

### Synthesis of compound 1

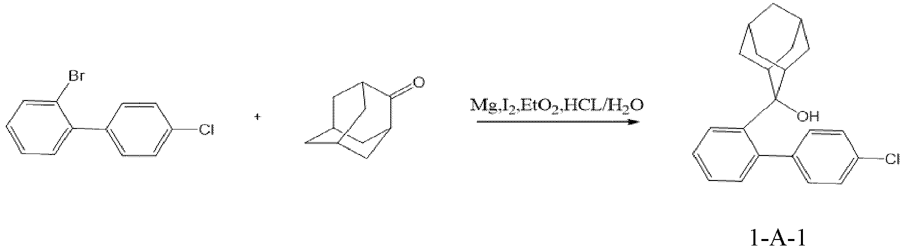

The magnesium strip (13.54g, 564mmol) and ether (100mL) were added into a round bottom flask dried under the nitrogen atmosphere, and iodine (100mg) was added. Then, the solution 2'-bromo-4-chlorobiphenyl (50.00g, 188.0mmol) of ether (200mL) was slowly added dropwise into the reaction mixture, the resulted mixture was raised to 35 °C after dropping and stirred for 3 hours; and then the reaction solution was reduced to 0 °C. A solution of amantadone (42.36g, 282.07mmol) in ether (200mL) was added dropwise to the reaction mixture, and the resulted mixture was heated to 35 °C after dropping. After stirred for 6 hours, the reaction solution was cooled to room temperature, then 5% hydrochloric acid was added to it until pH < 7. The mixture was stirred for 1 hour, then ether (200mL) was added for extraction. The combined organic phases were dried over anhydrous magnesium sulfate and filtered. The filtrate was concentrated in a vacuo to obtain a crude product. The crude product was purified by silica gel column chromatography and eluted with a mixture of ethyl acetate / n-heptane (1/2) to obtain intermediate 1-A-1 as a white solid (43g, yield 68.25%).

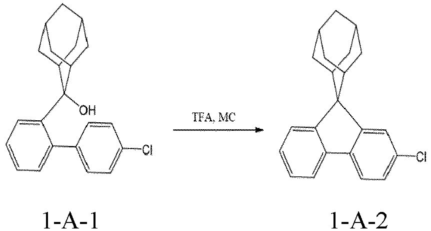

Intermediate 1-A-1 (43g, 126.8mmol), trifluoroacetic acid (36.93g, 380.6mmol) and dichloromethane (300mL) were added into a round bottom flask and the mixture was stirred under the nitrogen atmosphere for 2 hours; then, sodium hydroxide aqueous solution was added into the reaction solution until pH = 8. The separated organic phase was dried over anhydrous magnesium sulfate, and then concentrated in a vacuo to obtain a crude product. The crude product was purified by silica gel column chromatography and eluted with a mixture of dichloromethane and n-heptane (1/2) to obtain intermediate 1-A-2 as a white solid (39.2g, yield 96.3%).

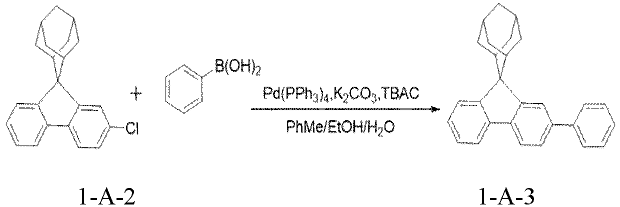

Intermediate 1-A-2 (39.20g, 122.17mmol), phenylboric acid (14.89g, 122.17mmol), tetra (triphenylphosphine) palladium (7.05g, 6.11mmol), potassium carbonate (33.77g, 244.34mmol), tetrabutylammonium chloride (1.39g, 6.11mmol), toluene (320mL), ethanol (160mL) and deionized water (80mL) were added into a round bottom flask, the reaction mixture was heated to 78°C under the nitrogen atmosphere and stirred for 18 hours. The reaction solution was cooled to room temperature, toluene (300mL) was added for extraction. The combined organic phases were dried with anhydrous magnesium sulfate, and filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain intermediate I-A-3 as a white solid (40.73g, yield 92%).

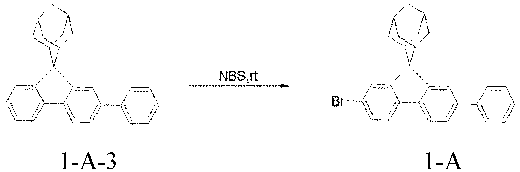

The intermediates 1-A-3 (40.73g, 112.35mmol), N-bromosuccinimide (23.99g, 134.82mmol) and dichloromethane (400mL) were added into a round bottom flask and stirred at room temperature under the nitrogen atmosphere for 2 hours. The separated organic phase was dried over anhydrous magnesium sulfate, filtered, and the filtrate was concentrated in a vacuo to obtain intermediate 1-A as a white solid (42.15g, yield 85%).

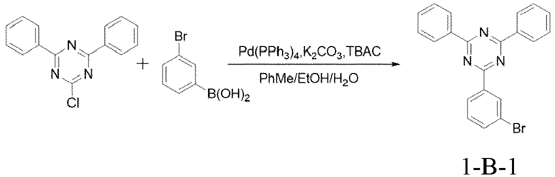

2-chloro-4,6-diphenyl-1,3,5-triazine (10.00g, 37.35mmol), m-bromophenylboric acid (7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethyl alcohol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction, the combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain intermediate 1-B-1 as a white solid (11.50g, yield 80%).

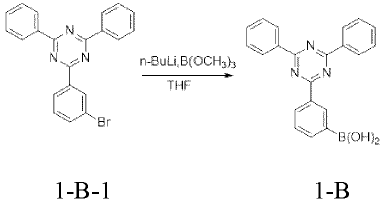

Intermediate 1-B-1 (11.50g, 29.61mmol) and THF (100mL) were added into a 250mL three-mouth bottle, then a solution of n-butyl lithium (1.99g, 31.09 mmol) was added dropwise at - 78°C. After dropwise addition, the solution was thermally insulated and stirred for 1 h, and then trimethyl borate (4.61g, 44.42mmol) was added dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-B as a white solid (8.66g, yield 83%).

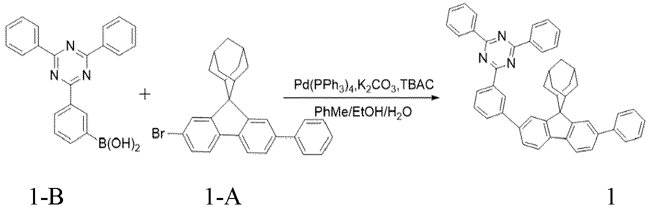

Intermediate 1-B (3.36g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the reaction mixture was heated to 78°C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 1 (5.1g, yield 80%). Mass spectrum: m/z=669.8 (M+H)⁺.

### Synthesis of compound 2:

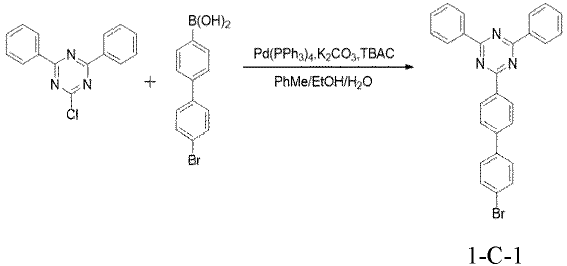

2-chloro-4,6-diphenyl-1,3,5-triazine (10.00g, 37.35mmol), 4'-bromo-4-biphenylboric acid(10.34g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction, the combined organic phases were dried with anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain intermediate 1-C-1 as a white solid (13.50g, yield 78%).

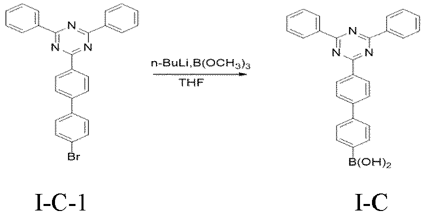

Intermediate 1-C-1 (13.50g, 29.07mmol) and THF (100mL) were added into a 250mL three-mouth bottle, and n-butyl lithium (1.99g, 31.09mmol) was added dropwise at - 78°C. After dropwise addition, the solution was thermally insulated and stirred for 1 h. Then trimethyl borate was added (4.61g, 44.42mmol) dropwise. The resulted mixture was stirred for 1h, then moved to room temperature and stirred overnight. Hydrochloric acid (2mol/L) was added to adjust the pH to neutral, filtered to obtain white crude product, and the crude product was stirred with n-heptane to obtain intermediate 1-C as a white solid (8.73g, yield 70%).

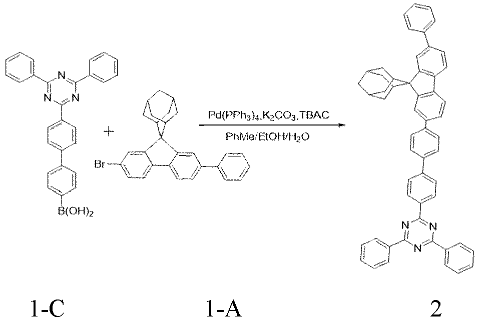

Intermediate 1-C (4.08g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the reaction mixture was heated to 78°C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 2 (5.87g, yield 83%). Mass spectrum: m/z=745.9(M+H)⁺.

### Synthesis of compound 3

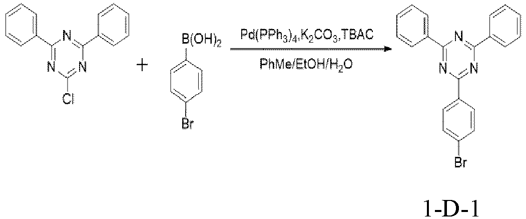

2-chloro-4,6-diphenyl-1,3,5-triazine (10.00g, 37.35mmol), P-bromophenylboric acid(7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the reaction was heated to 78 °C under the nitrogen atmosphere, and stirred for 10 hours. The reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-D-1 as a white solid (11.89g, yield 82%).

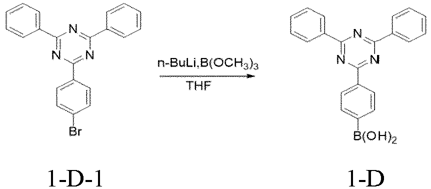

Intermediate 1-D-1 (11.89g, 30.62mmol) and THF (100mL) were added into a 250mL three-mouth bottle, then a solution of n-butyl lithium (1.99g, 31.09mmol) was added dropwise at - 78°C. After dropwise addition, the solution was thermally insulated and stirred for 1 h, and then trimethyl borate (4.61g, 44.42mmol) was added dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-D as a white solid (8.54g, yield 79%).

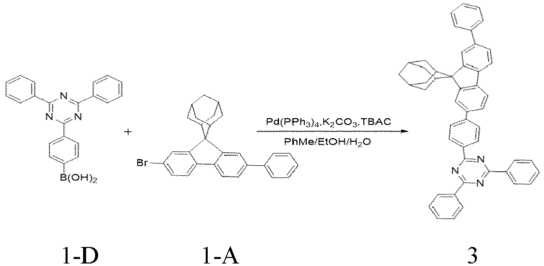

Intermediate 1-D (3.35g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the reaction mixture was heated to 78°C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 3 (5.35g, yield 84%). Mass spectrum: m/z=669.8 (M+H)⁺.

### Synthesis of compound 4

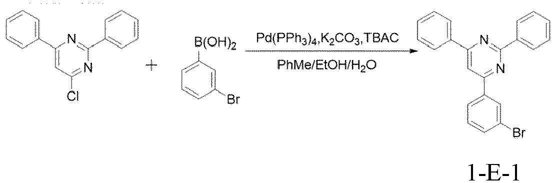

4-chloro-2.6-diphenylpyrimidine (10.00g, 37.49mmol), m-bromophenylboric acid (7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction. The combined organic phases were dried with anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain intermediate 1-E-1 as a white solid (12.47g, yield 86%).

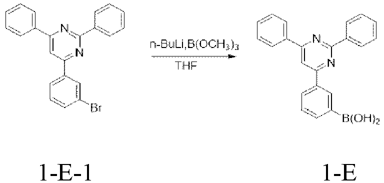

Intermediate 1-E-1 (12.47g, 32.20mmol) and THF (100mL) were added into a 250mL three-mouth bottle, n-butyl lithium (1.99g, 31.09mmol) was added dropwise at - 78°C. After dropwise addition, the solution was thermally insulated and stirred for 1 h. Then trimethyl borate (4.61g, 44.42mmol) was added dropwise. The resulted mixture was stirred for 1h, then moved to room temperature and stirred overnight. Hydrochloric acid (2mol/L) was added to adjust the pH to neutral, filtered to obtain white crude product, and the crude product was stirred with n-heptane to obtain intermediate 1-E as a white solid (8.51g, yield 75%).

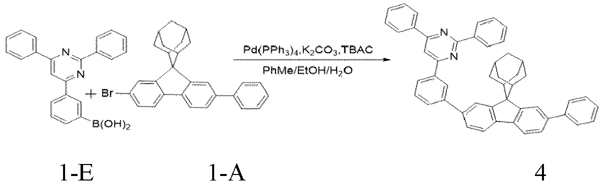

Intermediate 1-E (3.34g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78 °C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 4 (5.34g, yield 84%) Mass spectrum: m/z=668.8(M+H)⁺.

### Synthesis of compound 5

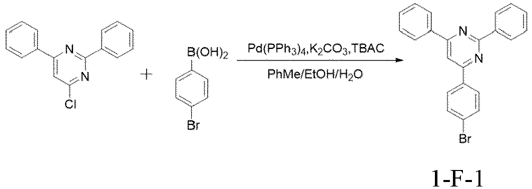

4-chloro-2.6-diphenylpyrimidine (10.00g, 37.49mmol), 4'-bromo-4-biphenylboric acid(10.34g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, the reaction mixture was heated to 78°C under the nitrogen atmosphere and stirred for 10 hours. the reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-F-1 as a white solid (12.18g, yield 84%).

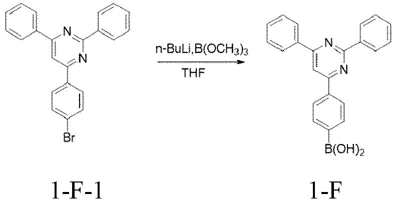

Intermediate 1-F-1 (12.18g, 31.45mmol) and THF (100mL) was added into a 250mL three-mouth bottle, and n-butyl lithium (1.99g, 31.09mmol) was added dropwise at -78°C. After dropwise addition, the solution was thermally insulated and stirred for 1 h, and then trimethyl borate (4.61g, 44.42mmol) was added dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-F as a white solid (7.68g, yield 70%).

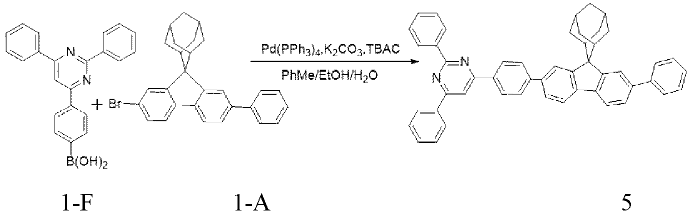

Intermediate 1-F (3.34g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the reaction mixture was heated 78°C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 5 (5.40g, yield 85%). Mass spectrum: m/z=668.8(M+H)⁺.

### Synthesis of compound 6

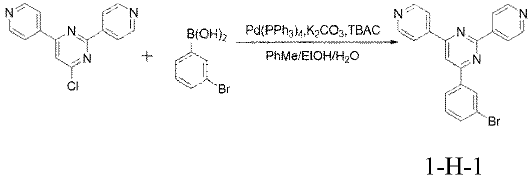

4-chloro-2.6-bis (pyridine-2-yl) pyrimidine (10.00g, 37.21mmol), m-bromophenylboric acid (7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the reaction mixture was heated to 78°C under the nitrogen atmosphere, stirred for 10 hours. Then the reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-H-1 as a white solid (12.16g, yield 84%).

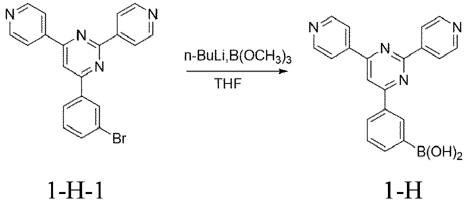

Intermediate 1-H-1 (12.16g, 31.23mmol) and THF (100mL) were added into a mL 250mL three-mouth bottle , n-butyl lithium (1.99g, 31.09mmol) was added dropwise at -78°C. After dropwise addition, the solution was thermally insulated and stirred for 1 h. Then trimethyl borate (4.61g, 44.42mmol) was then added dropwise. The resulted mixture was stirred for 1h, then moved to room temperature and stirred overnight. Hydrochloric acid (2mol/L) was added to adjust the pH to neutral, filtered to obtain white crude product, and the crude product was stirred with n-heptane to obtain intermediate 1-H as a white solid (8.20g, yield 71%).

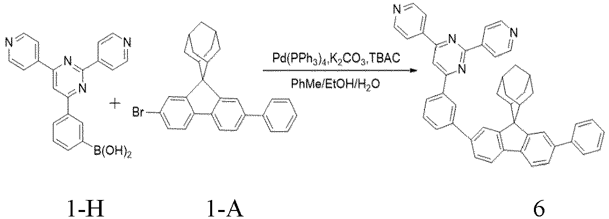

Intermediate 1-H (3.36g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours. Then the reaction solution was cooled to room temperature, toluene (200mL) was added for extraction. The combined organic phases were dried with anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 6 (5.42g, yield 85%). Mass spectrum: m/z=670.8(M+H)⁺.

### Synthesis of compound 7

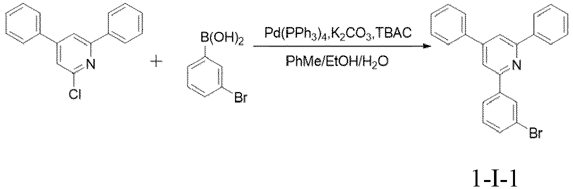

2-chloro-4.6-diphenylpyridine (10.00g, 37.21mmol), m-bromophenylboric acid (7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction. The combined organic phases were dried with anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain intermediate 1-1-1 as a white solid (11.91g, yield 82%).

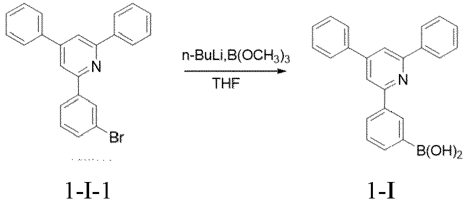

Intermediate 1-I-1 (11.91g, 30.83mmol) and THF (100mL) were added into a 250mL three-mouth bottle, then a solution of n-butyl lithium (1.99g, 31.09mmol) was added dropwise at -78°C. After dropwise addition, the solution was thermally insulated and stirred for 1 h, and then trimethyl borate (4.61g, 44.42mmol) was added dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-1 as a white solid (7.47g, yield 69%).

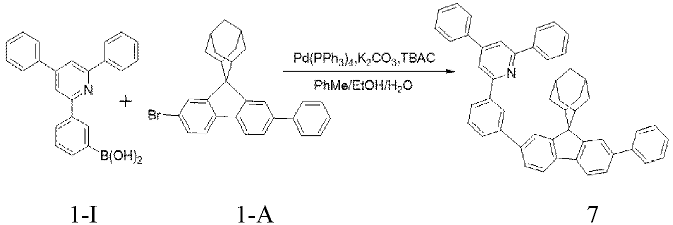

Intermediate 1-I (3.33g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours. Then the reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 7 (5.08g, yield 85%). Mass spectrum: m/z=667.7(M+H)⁺.

### Synthesis of compound 8

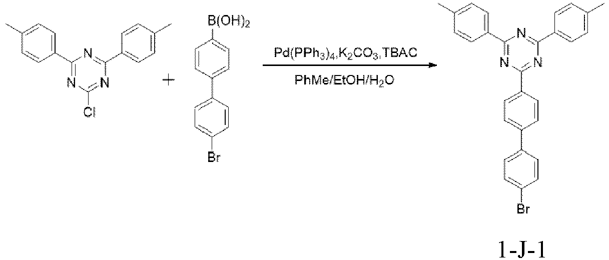

2-chloro-4.6-bis-p-tolyl-1.3.5-triazine(10.00g, 33.81mmol), 4'-bromo-4-biphenylboric acid (10.34g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-J-1 as a white solid (13.81g, yield 83%).

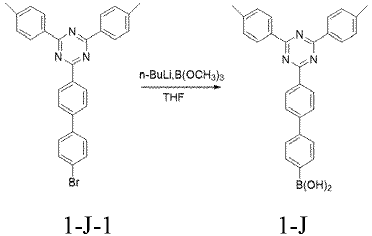

Intermediate 1-J-1 (13.81g, 28.04mmol) and THF (100mL) were added into a 250mL three-mouth bottle , a solution of n-butyl lithium (1.99g, 31.09mmol) was added dropwise at - 78°C. dropwise addition, the solution was thermally insulated and stirred for 1 h, and then trimethyl borate (4.61g, 44.42mmol) was added dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-J as a white solid (8.71g, yield 68%).

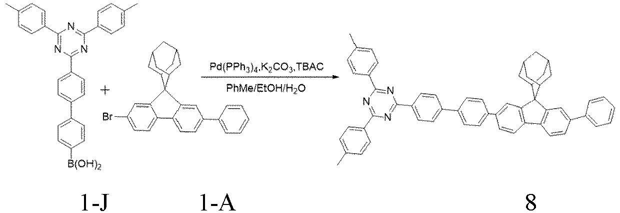

Intermediate 1-J (4.35g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours. The reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 8 (5.96g, yield 81%). Mass spectrum: m/z=774.0(M+H)⁺.

### Synthesis of compound 9:

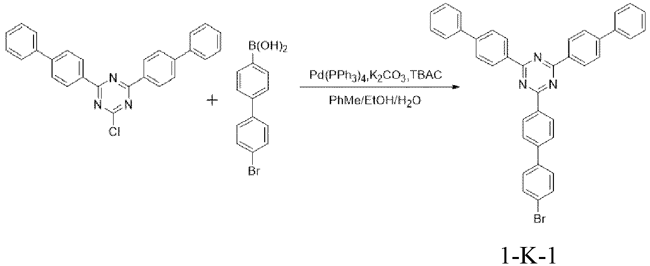

2.4-bis ([1.1'-biphenyl]-4-yl)-6-chloro-1,3,5-triazine (10.00g, 23.81mmol), 4'-bromo-4-biphenylboric acid (10.34g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask. The mixture was heated to 78°C under the nitrogen atmosphere and stirred for 10 hours; then the reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-K-1 as a obtain white (12.18g, yield 83%).

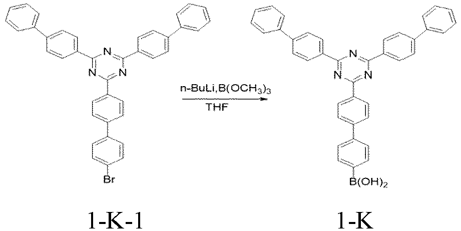

Intermediate 1-K-1 (12.18g, 19.75mmol) and THF (100mL) were added into a 250mL three mouth bottle, a solution of n-butyl lithium (1.99g, 31.09mmol) was added dropwise at - 78°C. After dropwise addition, the solution was thermally insulated and stirred for 1 h. Then trimethyl borate (4.61g, 44.42mmol) was added dropwise. The resulted mixture was stirred for 1h, then moved to room temperature and stirred overnight. Hydrochloric acid (2mol/L) was added to adjust the pH to neutral, filtered to obtain white crude product, and the crude product was stirred with n-heptane to obtain intermediate 1-K as a white solid (7.69g, yield 67%).

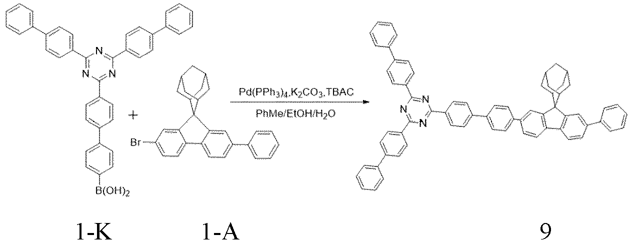

Intermediate 1-K (5.53g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours; then the reaction solution was cooled to room temperature, toluene (200mL) was added for extraction. The combined organic phases were dried with anhydrous magnesium sulfate and filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 9 (6.83g, yield 80%). Mass spectrum: m/z=898.1(M+H)⁺.

### Synthesis of compound 10:

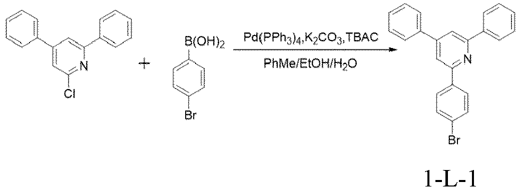

2-chloro-4.6-diphenylpyridine (10.00g, 37.21mmol), p-bromophenylboric acid (7.50g, 37.35mmol), tetra (triphenylphosphine) palladium (2.15g, 1.87mmol), potassium carbonate (10.32g, 74.70mmol), tetrabutylammonium chloride (0.43g, 1.86mmol), toluene (80mL), ethanol (40mL) and deionized water (20mL) were added into a round bottom flask, and the mixture was heated to 78°C under the nitrogen atmosphere, stirred for 10 hours. Then the reaction solution was cooled to room temperature, toluene (300mL) was added for extraction. The combined organic phases were dried with anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate system to obtain intermediate 1-L-1 as a white solid (11.91g, yield 82%).

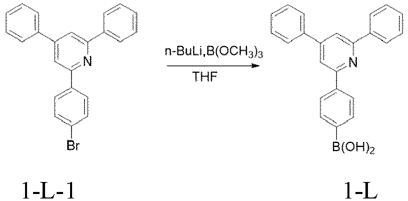

Intermediate 1-L-1 (11.91g, 30.83mmol) and THF (100mL) were added into a 250mL three-mouth bottle , a solution of n-butyl lithium (1.99g, 31.09mmol) was added dropwise at - 78°C. After dropwise addition, the solution was thermally insulated and stirred for 1 h. Then trimethyl borate (4.61g, 44.42mmol) was added dropwise. The resulted mixture was stirred for 1h, then moved to room temperature and stirred overnight. Hydrochloric acid (2mol/L) was added to adjust the pH to neutral, filtered to obtain white crude product, and the crude product was stirred with n-heptane to obtain intermediate 1-L as a white solid (7.47g, yield 69%).

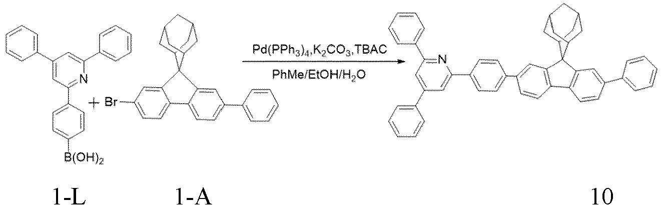

Intermediate 1-L (3.33g, 9.51mmol), intermediate 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours; the reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 10 (5.11g, yield 86%). Mass spectrum: m/z=667.7(M+H)⁺.

### Synthesis of compound 11

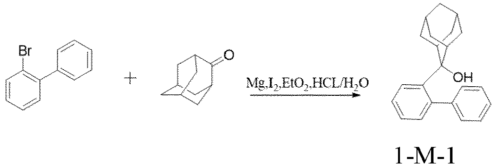

The magnesium strip (13.54g, 564mmol) and ether (100mL) were added into a round bottom flask dried under the nitrogen atmosphere, and iodine (100mg) was added; then, the solution of 2-bromobiphenyl (25.00g, 94mmol) in ether (200mL) was slowly added dropwise into the reaction mixture, the mixture was heated to 35 °C after dropping, and stirred for 3 hours; and then the reaction solution was reduced to 0°C. A solution of amantadone (42.36g, 282.07mmol) in ether (200mL) was added dropwise to the reaction mixture, and the resulted mixture was heated to 35 °C after dropping. After stirred for 6 hours, the reaction solution was cooled to room temperature, then 5% hydrochloric acid was added to it until pH < 7. The mixture was stirred for 1 hour, then ether (200mL) was added for extraction. The combined organic phases were dried over anhydrous magnesium sulfate and filtered. The filtrate was concentrated in a vacuo to obtain a crude product. The crude product was purified by silica gel column chromatography and eluted with a mixture of ethyl acetate and n-heptane (1/2)to obtain intermediate 1-M-1 as a white solid (27.06g, yield 85.03%).

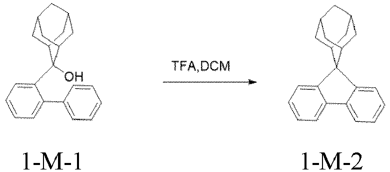

Intermediate 1-M-1 (27.06g, 88.97mmol), trifluoroacetic acid (46.90g, 483.36mmol) and dichloromethane (300mL) were added into a round bottom flask and the mixture was stirred under the nitrogen atmosphere for 2 hours; then, sodium hydroxide aqueous solution was added to the reaction solution until pH = 8. The separated organic phase was dried over anhydrous magnesium sulfate, and then concentrated in a vacuo to obtain a crude product. The crude product was purified by silica gel column chromatography and eluted with a mixture of dichloromethane and n-heptane (1/2) to obtain intermediate 1-M-2 as a white solid (25.40g, yield 99.70%).

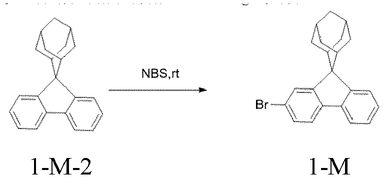

The intermediates 1-M-2 (25.40g, 88.67mmol), N-bromosuccinimide (30.46g, 171.22mmol) and dichloromethane (400mL) were added into a round bottom flask and the reaction mixture was stirred at room temperature under nitrogen atmosphere for 2 hours. The separated organic phase was dried over anhydrous magnesium sulfate, filtered, and the filtrate was concentrated in a vacuo to obtain intermediate 1-M as a white solid (31.75g, yield 98.04%).

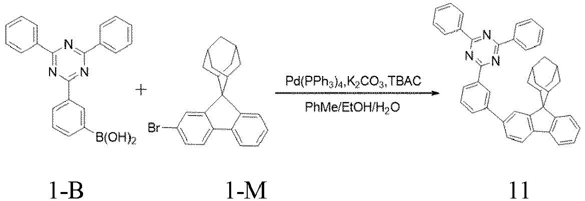

Intermediate 1-B (3.36g, 9.51mmol), intermediate 1-M (4.65g, 12.74mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours. Then the reaction solution was cooled to room temperature, toluene (200mL) was added for extraction. The combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate system to obtain compound 11 (6.21g, yield 82%). Mass spectrum: m/z=593.7(M+H)⁺. The hydrogen spectrum of compound 11 is: ¹H NMR (CDCl₃, 400MHz): 9.16 (s, 1H), 8.81 (d, 4H), 8.77 (d, 1H), 8.55 (s, 1H), 8.16 (d, 1H), 7.93 (d, 2H), 7.86 (d, 1H), 7.77 (d, 1H), 7.69 (t, 1H), 7.64 (t, 2H), 7.59 (t, 4H), 7.42 (t, 1H), 7.33 (t, 1H), 3.17 (d, 2H), 3.00 (d, 2H), 2.36 (s, 1H), 2.27 (s, 1H), 2.07 (s, 2H), 1.93 (d, 2H), 1.86 (d, 2H), 1.74 (s, 2H).

### Synthesis of compound 12

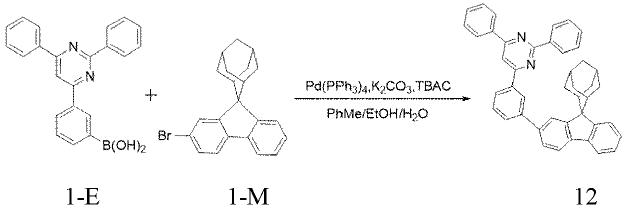

Intermediate 1-E (3.34g, 9.51mmol), intermediate 1-M (4.66g, 12.77mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours; the reaction solution was cooled to room temperature, toluene (200mL) was added for extraction. The combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 12 (6.10g, yield 81%). Mass spectrum: m/z=592.7(M+H)⁺.

### Synthesis of compound 13

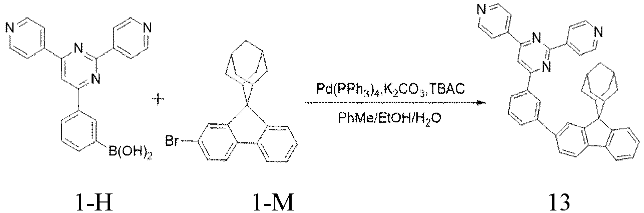

The intermediates 1-H (3.36g, 9.51mmol), 1-M (4.64g, 12.70mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added to a round bottom flask, the mixture was heated to 78°C under the nitrogen atmosphere and stirred for 10 hours; the reaction solution was cooled to room temperature, toluene (200mL) was added for extraction. The combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 13 (6.34g, yield 83%). Mass spectrum: m/z=594.7(M+H)⁺.

### Synthesis of compound 14

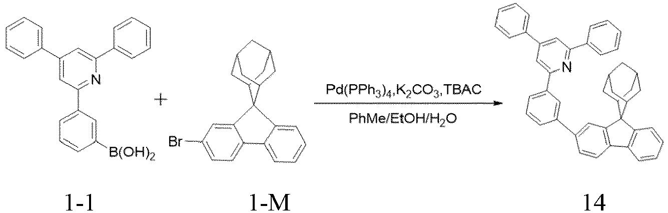

Intermediate 1-I (3.33g, 9.51mmol), intermediate 1-M (4.68g, 12.81mmol) 1-A (4.2g, 9.51mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethyl alcohol (16mL) and deionized water (8mL) were added into a round bottom flask, and the mixture was heated to 78°C under the nitrogen atmosphere, stirred for 10 hours; the reaction solution was cooled to room temperature, toluene (200mL) was added for extraction. The combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 14 (6.29g, yield 83%). Mass spectrum: m/z=591.8(M+H)⁺.

### Synthesis of compound 15

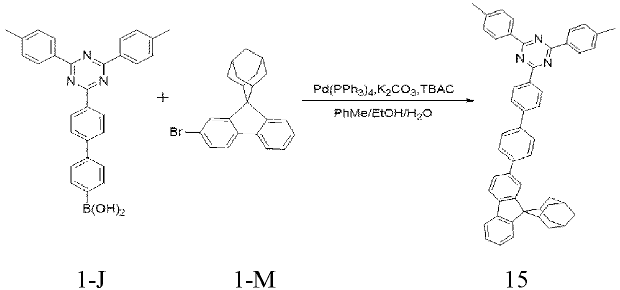

Intermediate 1-J (4.35g, 9.51mmol), intermediate 1-M (3.59g, 9.83mmol), tetra (triphenylphosphine) palladium (0.55g, 0.47mmol), potassium carbonate (2.63g, 19.02mmol), tetrabutylammonium chloride (0.11g, 0.47mmol), toluene (30mL), ethanol (16mL) and deionized water (8mL) were added into a round bottom flask, the mixture was heated to 78°C under the nitrogen atmosphere and stirred for 10 hours; the reaction solution was cooled to room temperature, toluene (200mL) was added for extraction. The combined organic phases were dried over anhydrous magnesium sulfate, filtered. The filtrate was concentrated in a vacuo to obtain a residue. The residue was purified by silica column chromatography and eluted with n-heptane to obtain a crude product. The crude product was purified by recrystallization using a mixture of dichloromethane and ethyl acetate to obtain compound 15 (5.69g, yield 83%). Mass spectrum: m/z=697.9(M+H)⁺.

### Synthesis of compound 16:

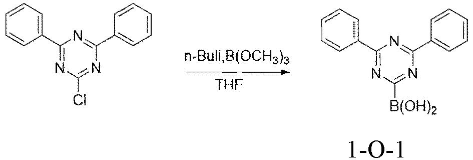

2-chloro-4,6-diphenyl-1,3,5-triazine (30.00g, 112.06mmol) and THF (300mL) were added into a 500mL three-mouth bottle , a solution of n-butyl lithium (7.54g, 117.66mmol) was added dropwise at -78°C. After dropwise addition, the solution was thermally insulated and stirred for 1 h, and then trimethyl borate (17.46g, 168.09mmol) was added dropwise. The resulted mixture was stirred was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-O-1 as a white solid (21.73g, yield 70%).

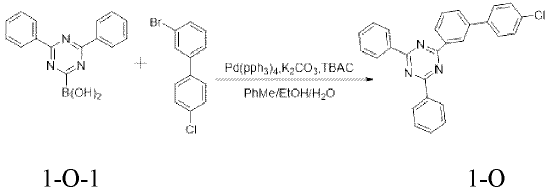

The intermediates 1-O-1 (5.0g, 18.04mmol), 4'-chloro-3-bromobiphenyl (5.31g, 19.84mmol), tetra (triphenylphosphine) palladium (0.41g, 0.36mmol), potassium carbonate (4.98g, 36.08mmol), tetrabutylammonium chloride (0.25g, 0.90mmol), toluene (80mL), ethanol (40mL) and deionized water (20mL) were added into a round bottom flask, the reaction mixture was heated to 78°C under the nitrogen atmosphere and stirred for 10 hours; the reaction solution was cooled to room temperature, toluene (300mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain intermediate 1-O as a white solid (6.20g, yield 82%).

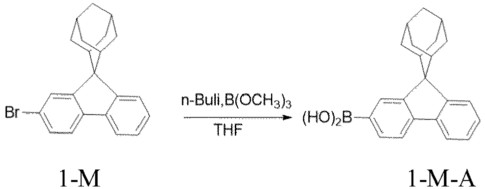

Intermediate 1-M (8.5g, 23.27mmol) and THF (300mL) were added into a 500mL three-mouth bottle, and a solution of n-butyl lithium (1.56g, 24.43mmol) was added dropwise at -78°C. After dropwise addition, the solution was thermally insulated and stirred for 1 h, then trimethyl borate was added (3.63g, 34.90mmol) dropwise. The resulted mixture was stirred for 1h, then moved to room temperature and stirred overnight. Hydrochloric acid (2mol / L) was added to adjust the pH until neutral, filtered to obtain white crude product, and the crude product was stirred with n-heptane to obtain intermediate 1-M-A (6.05g, yield 78%).

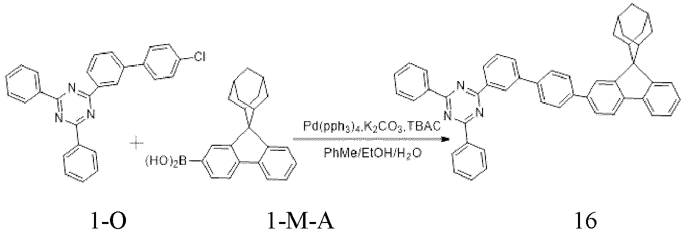

Intermediate 1-O (1.50g, 3.57mmol), intermediate 1-M-A (1.30g, 3.92mmol), tetra (triphenylphosphine) palladium (0.082g, 0.07mmol), potassium carbonate (0.98g, 7.14mmol), tetrabutylammonium chloride (0.05g, 0.18mmol), toluene (12mL), ethanol (6mL) and deionized water (3mL) were added into a round bottom flask, the mixture was heated to 78°C under the nitrogen atmosphere and stirred for 5 hours; the reaction solution was cooled to room temperature, toluene (200mL) was added for extraction. The separated organic phase was dried over anhydrous MgSO₄ and filtered. The filtrate was concentrated in a vacuo to obtain a residue. The residue was purified by silica column chromatography and eluted with n-heptane, and then the crude product was purified by recrystallized using a mixture of dichloromethane and ethyl acetate to obtain compound 16 (1.86g, yield 78%). Mass spectrum: m/z=669.9(M+H)⁺. The hydrogen spectrum of compound 16 is: ¹H NMR (CDCl₃, 400MHz): 9.07 (s, 1H), 8.82-8.78 (m, 5H), 8.41 (s, 1H), 8.15 (d, 1H), 7.92-7.87 (m, 4H), 7.85 (d, 1H), 7.82 (d, 2H), 7.70-7.68 (m, 2H), 7.64-7.59 (m, 6H), 7.41 (t, 1H), 7.32 (t, 1H), 3.06 (d, 2H), 2.98 (d, 2H), 2.29 (s, 1H), 2.24 (s, 1H), 2.03 (s, 2H), 1.88-1.82 (m, 4H), 1.71 (s, 2H).

### Synthesis of compound 17:

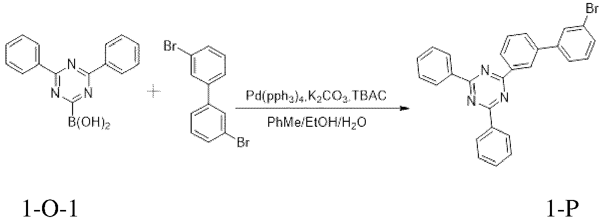

The synthesis of intermediate 1-P was basically the same as that of intermediate 1-O and the post-treatment method, except that 3, 3' dibromo biphenyl (5.31g, 19.84mmol) was used instead of 4'-chloro-3-bromobiphenyl (5.62g, 18.04mmol) to obtain intermediate 1-P (6.11g, yield 73%).

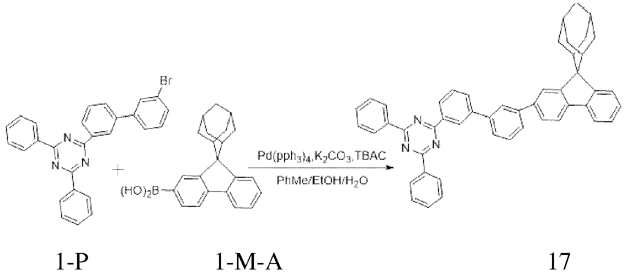

The synthesis of compound 17 was basically consistent with the synthesis process and post-treatment method of compound 16, except that intermediate 1-P (1.50g, 3.23mmol) was used to replace intermediate 1-O (1.50g, 3.57mmol) to obtain compound 17 (1.62g, yield 75%). Mass spectrum: m/z=669.9(M+H)⁺. The hydrogen spectrum of compound 17 is: ¹H NMR (CDCl₃, 400MHz): 9.05 (s, 1H), 8.80 (d, 5H), 8.40 (s, 1H), 8.14 (d, 1H), 7.97 (s, 1H), 7.90 (t, 2H), 7.84 (d, 1H), 7.75 (d, 1H), 7.71-7.69 (m, 3H), 7.66-7.57 (m, 7H), 7.40 (t, 1H), 7.31 (t, 1H), 3.04 (d, 2H), 2.97 (d, 2H), 2.23 (s, 2H), 1.97 (s, 2H), 1.82 (t, 4H), 1.70(s, 2H).

Other compounds of the invention can be prepared with reference to the synthesis process of the above compounds.

Next, taking an organic electroluminescent device as an example, the organic electroluminescent device of the present disclosure will be described in detail through an example. However, the following examples are only examples of the present disclosure and do not limit the present disclosure.

### Manufacturing and Evaluation Examples of Organic Electroluminescent Devices

### Manufacturing of blue organic electroluminescent devices

### Example 1:

Anode 1 was prepared by the following process: ITO substrate (manufactured by Kangning) was cut with ITO thickness of 1500 Å into 40mm(length)×40mm(width)×0.7mm(thickness). The photolithography process was used to prepare the top emission experimental substrate with cathode lap area, anode and insulating layer pattern. The surface was treated with ultraviolet ozone and O₂:N₂ plasma to increase the work function of anode (experimental substrate) and clean the experimental substrate.

m-MTDATA(4,4',4"-tris (N-3-methylphenyl-N-phenylamino) trianiline) was vacuum evaporated on the experimental substrate (anode) to form a hole injection layer (HIL) with a thickness of 100Å, and NPB(N,N'-diphenyl-N,N'-(1-naphthyl)-1,1'-biphenyl-4,4'-diamine) was vacuum evaporated on the hole injection layer to form a hole transport layer (HTL) with a thickness of 800Å.

TCTA(4,4',4"- tris(carbazole-9-yl) triphenylamine) was evaporated on the hole transport layer to form an electron blocking layer 32 (EBL) with a thickness of 300Å.

α, β-AND was used as the main body and doped with 4,4'-(3,8-diphenylpyrene-1,6-diyl) bis (N,N-diphenylaniline). The main body and dopant form a light emitting layer (EML) with a thickness of 220Å at a membrane thickness ratio of 100:3.

Compound 1 on the light emitting layer was vaporated to form an electron transport layer (ETL) with a thickness of 300Å, evaporate Yb on the electron transport layer to form an electron injection layer (EIL) with a thickness of 15Å, then magnesium (Mg) and silver (Ag) was mixed at an evaporation rate of 1:9, vacuum evaporated on the electron injection layer to form a cathode with a thickness of 120Å.

In addition, CP-01 (N-(4-(9H-carbazole-9-yl) phenyl)-4'-(9H-carbazole-9-yl)-N-phenyl-[1,1'-biphenyl]-4-amine) with a thickness of 650Å was evaporated on the cathode to form a covering layer(CPL), so as to complete the manufacture of organic light-emitting devices.

### Examples 2-17

In addition to using the compounds shown in Table 1 respectively when forming the electron transport layer (ETL), an organic electroluminescent device was fabricated by the same method as in Example 1.

### Comparative Example 1 - Comparative Example 3

In the Comparative Example 1 - Comparative Example 3, in addition to using Alq₃, TPBi, Bphen as the electron transport layer 35 instead of compound 1, the organic electroluminescent device was manufactured in the same way as in Example 1.

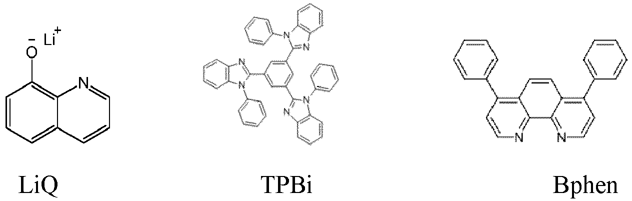

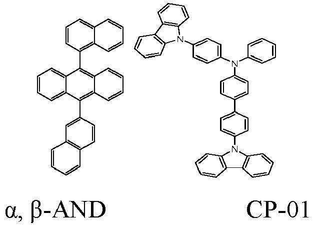

That is, Comparative Example 1 uses Alq₃ to manufacture organic electroluminescent devices, Comparative Example 2 uses TPBi to manufacture organic electroluminescent devices, and Comparative Example 3 uses Bphen to manufacture organic electroluminescent devices. See Table 1 for device performance.

Among them, IVL (current, voltage and brightness) data was compared by the test results at 10mA/cm², and the T95 lifetime was the test results at the current density of 15mA/cm².

**Table 1 Device Performance of Examples 1-17 and Comparative Examples 1-3**

| Examples | Compound | Working voltage Volt(V) | Luminous efficiency Cd/A | External quantum efficiency EQE (%) | T95 lifetime (h) | Chromaticity coordinates CIEy |
|---|---|---|---|---|---|---|
| Example 1 | Compound 1 | 4.01 | 6.3 | 13.0 | 268 | 0.047 |
| Example 2 | Compound 2 | 3.99 | 6.2 | 12.8 | 263 | 0.046 |
| Example 3 | Compound 3 | 3.95 | 6.5 | 13.5 | 275 | 0.046 |
| Example 4 | Compound 4 | 4.03 | 6.1 | 12.6 | 265 | 0.047 |
| Example 5 | Compound 5 | 4.01 | 6.4 | 13.3 | 254 | 0.047 |
| Example 6 | Compound 6 | 3.97 | 6.3 | 13.0 | 260 | 0.048 |
| Example 7 | Compound 7 | 4.01 | 6.3 | 13.1 | 259 | 0.047 |
| Example 8 | Compound 8 | 4.08 | 6.2 | 12.8 | 272 | 0.046 |
| Example 9 | Compound 9 | 3.98 | 6.4 | 13.4 | 254 | 0.048 |
| Example 10 | Compound 10 | 3.96 | 6.5 | 13.5 | 268 | 0.048 |
| Example 11 | Compound 11 | 4.00 | 6.1 | 12.6 | 252 | 0.047 |
| Example 12 | Compound 12 | 4.02 | 6.1 | 12.7 | 273 | 0.046 |
| Example 13 | Compound 13 | 3.94 | 6.3 | 13.1 | 261 | 0.048 |
| Example 14 | Compound 14 | 3.99 | 6.5 | 13.4 | 253 | 0.046 |
| Example 15 | Compound 15 | 4.01 | 6.4 | 13.4 | 255 | 0.047 |
| Example 16 | Compound 16 | 4.00 | 6.3 | 13.2 | 263 | 0.046 |
| Example 17 | Compound 17 | 3.98 | 6.5 | 13.1 | 265 | 0.048 |
| Comparative example 1 | Alq₃ | 4.28 | 5.2 | 10.7 | 155 | 0.047 |
| Comparative example 2 | TPBi | 4.24 | 4.5 | 9.3 | 136 | 0.047 |
| Comparative example 3 | Bphen | 4.23 | 5.5 | 11.4 | 170 | 0.047 |

It can be seen from the results of Table 1 above that compared with Comparative Example 1, Comparative Example 2 and Comparative Example 3 using well-known Alq3, TPBi and Bphen, organic electroluminescent devices prepared by compounds 1-17 as electron transport layer 35 (ETL) reduce the operating voltage by 8%; increased the luminous efficiency (Cd/A) by at least 10.9%; increased the external quantum efficiency by at least 10.7%; extended the service life by at least 82 hours and increased by at least 48%. Therefore, the performance of the organic electroluminescent device according to the example of the present disclosure is significantly improved.

For the organic electroluminescent device according to the example of the present disclosure, in 400(V/cm)^{1/2} electric field intensity, since the electron mobility of the compound used can reach more than 4*10⁻³cm²/V.s, the prepared organic electroluminescent device has high current efficiency; because the compound has a LUMO energy level more matched with the adjacent layer, the prepared organic electroluminescent device has a very low driving voltage. In addition, the adamantyl introduced into the compound increases the molecular weight of the material, reduces the molecular symmetry, improves the glass transition temperature and evaporation temperature of the material, controls the crystallinity of the material, so that the material has better physical and thermal stability, good durability and heat resistance when used in mass production, so as to significantly improve the service life of organic electroluminescent devices.

After considering the description and practicing the invention disclosed herein, those skilled in the art will easily think of other examples of the present disclosure. The present disclosure aims to cover any modification, use or adaptive change of the present disclosure, which follows the general principles of the present disclosure and includes the common general knowledge or frequently used technical means in the technical field not disclosed in the present disclosure.

## Claims

1. A compound, wherein the general structural formula of the compound is shown in formula 1: 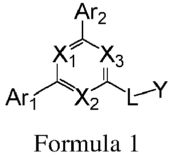 wherein Y is selected from: 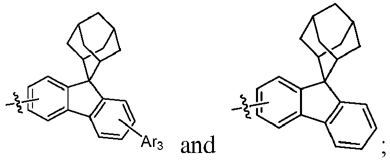
X₁ and X₂ are independently selected from carbon and nitrogen; X₃ is nitrogen;
L is selected from single bond, substituted or unsubstituted arylene with 6 to 30 carbon atoms, substituted or unsubstituted heteroarylene with 2 to 29 carbon atoms, substituted or unsubstituted arylalkylene with 7 to 30 carbon atoms, and substituted or unsubstituted heteroarylalkylene with 3 to 29 carbon atoms;
Ar₁, Ar₂ and Ar₃ are the same or different, and are independently selected from substituted or unsubstituted arylalkyl with 7 to 30 carbon atoms, substituted or unsubstituted heteroarylalkyl with 2 to 29 carbon atoms, substituted or unsubstituted aryl with 6 to 30 carbon atoms, and substituted or unsubstituted heteroaryl with 2 to 30 carbon atoms;
the substituents of Ar₁, Ar₂, Ar₃ and L are the same or different, and are independently selected from deuterium, cyano, nitro, halogen, hydroxyl, substituted or unsubstituted alkyl with 1 to 24 carbon atoms, substituted or unsubstituted cycloalkyl with 3 to 24 carbon atoms, substituted or unsubstituted alkenyl with 2 to 24 carbon atoms, substituted or unsubstituted alkynyl with 2 to 24 carbon atoms, substituted or unsubstituted heterocycloalkyl with 2 to 24 carbon atoms, substituted or unsubstituted arylalkyl with 7 to 24 carbon atoms, substituted or unsubstituted heteroarylalkyl with 2 to 24 carbon atoms, substituted or unsubstituted aryl with 6 to 24 carbon atoms, substituted or unsubstituted heteroaryl with 1 to 24 carbon atoms, substituted or unsubstituted alkoxy with 1 to 24 carbon atoms, substituted or unsubstituted alkylamino with 1 to 24 carbon atoms, substituted or unsubstituted arylamino with 6 to 24 carbon atoms, substituted or unsubstituted alkylthio with 1 to 24 carbon atoms, substituted or unsubstituted arylalkylamino with 7 to 24 carbon atoms, substituted or unsubstituted heteroarylamino with 1 to 24 carbon atoms, substituted or unsubstituted alkylsilyl with 1 to 24 carbon atoms, substituted or unsubstituted arylsilyl with 6 to 24 carbon atoms, substituted or unsubstituted aryloxy with 6 to 24 carbon atoms, and substituted or unsubstituted arylthio with 6 to 24 carbon atoms.

2. The compound according to claim 1, wherein L is selected from arylene with 6 to 25 ring-forming carbon atoms.

3. The compound according to claim 1 or 2, wherein L is selected from the group consisting of the following groups: 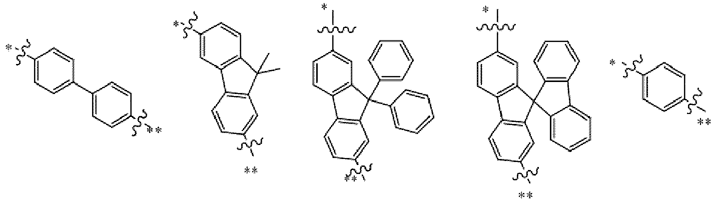 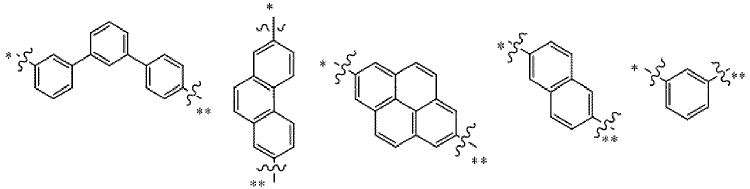 wherein * represents a linkage with the group of 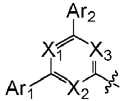 in formula 1; ** represents a linkage with the group of 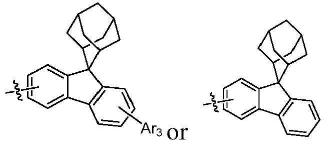 in formula 1.

4. The compound according to any one of claims 1 to 3, wherein Ar₁ and Ar₂ are the same or different, and are independently selected from substituted or unsubstituted aryl with 6 to 30 carbon atoms, and substituted or unsubstituted heteroaryl with 2 to 29 carbon atoms.

5. The compound according to any one of claims 1 to 4, wherein Ar₁ and Ar₂ are the same or different, and are independently selected from substituted or unsubstituted aryl with 6 to 12 ring-forming carbon atoms, and substituted or unsubstituted heteroaryl with 5 ring-forming carbon atoms.

6. The compound according to any one of claims 1 to 5, wherein Ar₁ and Ar₂ are the same or different, and are independently selected from the group formed by the following groups: 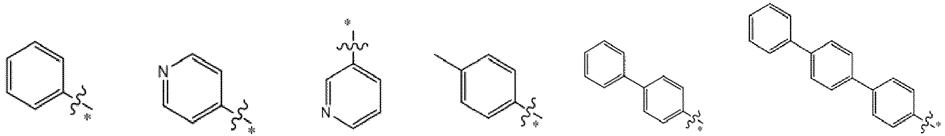 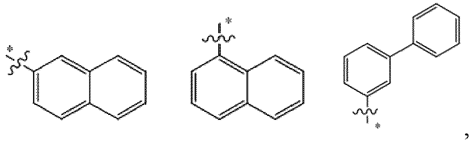 wherein * represents a linkage with the group of 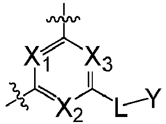 in formula 1.

7. The compound according to any one of claims 1 to 6, wherein the compound is selected from the group consisting of the following compounds:

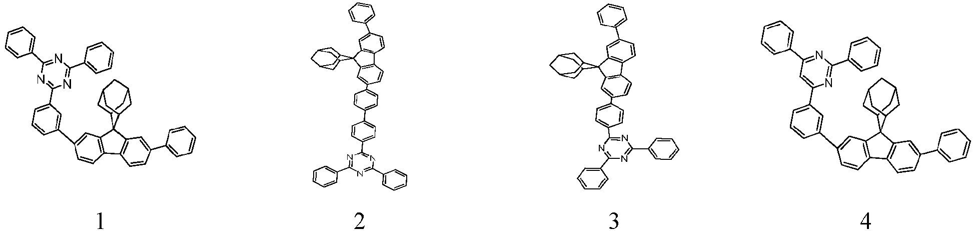

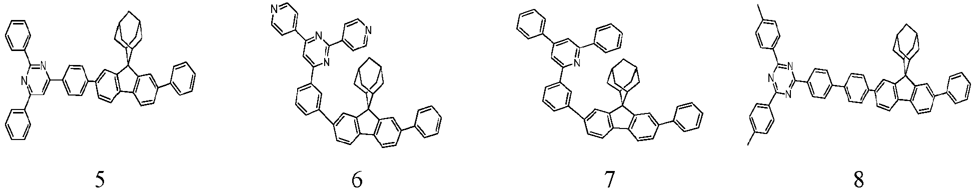

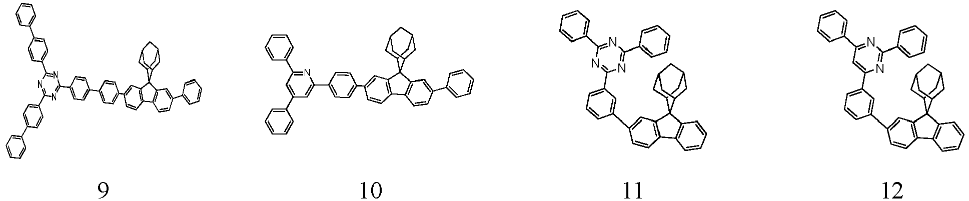

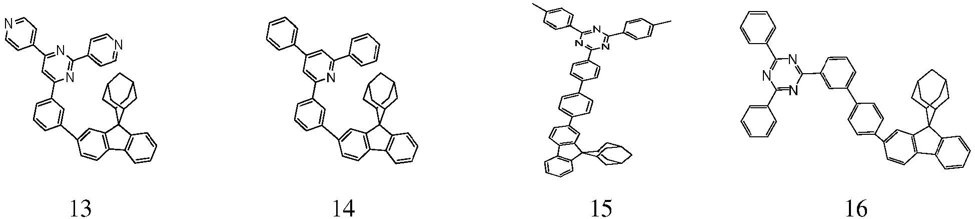

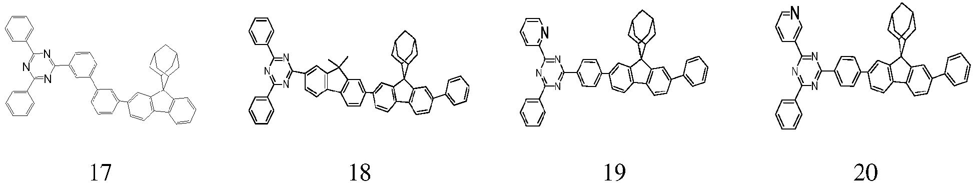

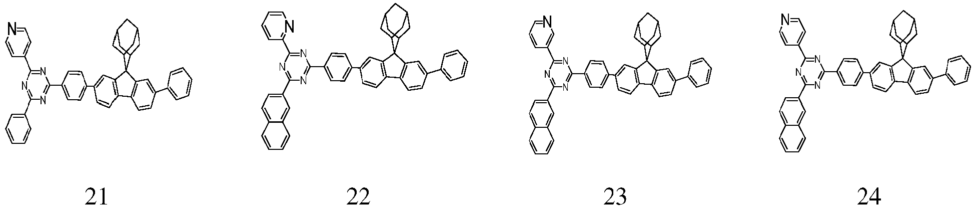

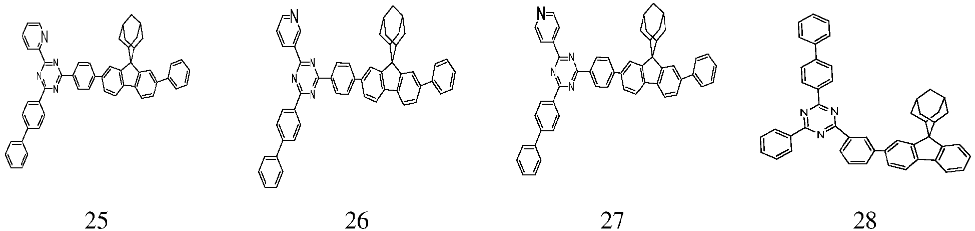

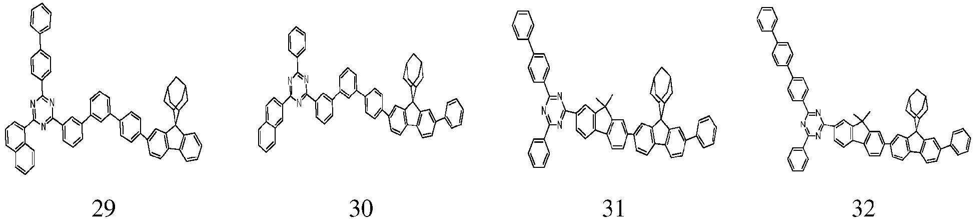

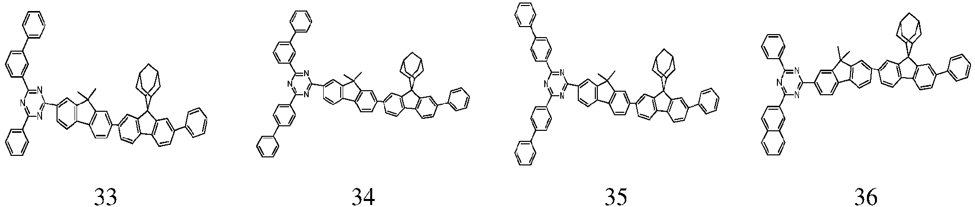

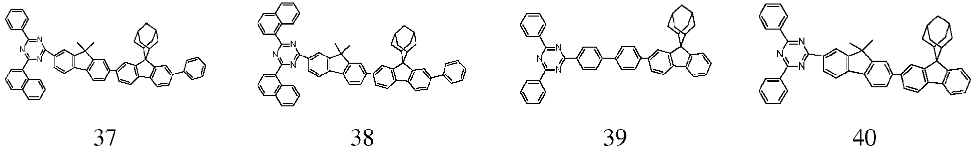

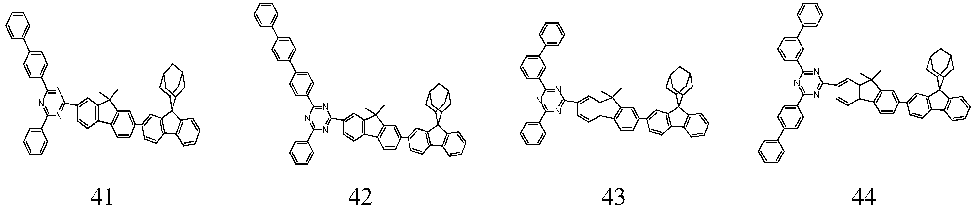

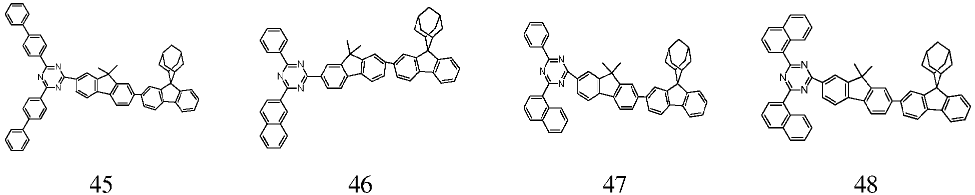

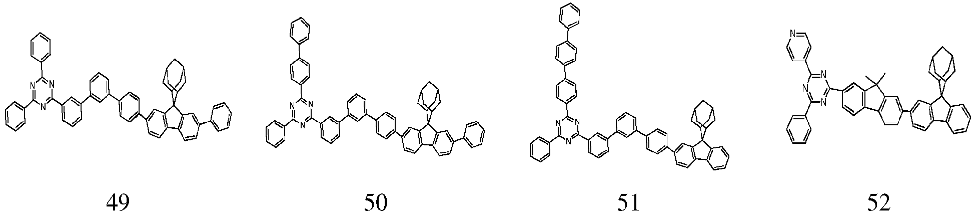

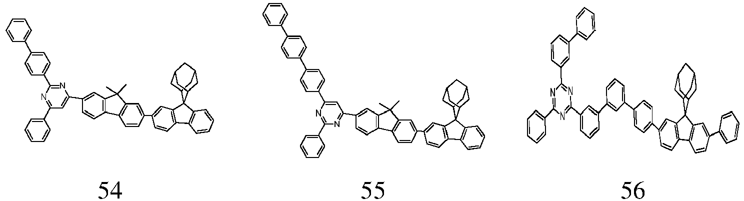

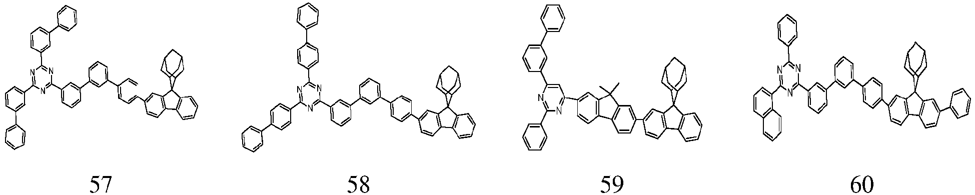

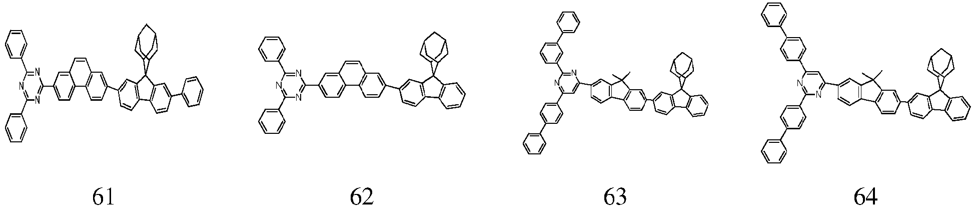

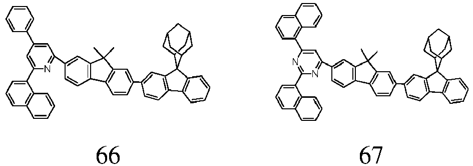

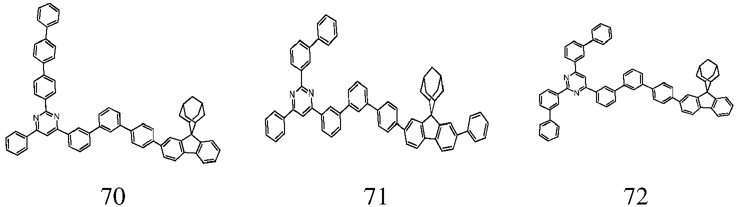

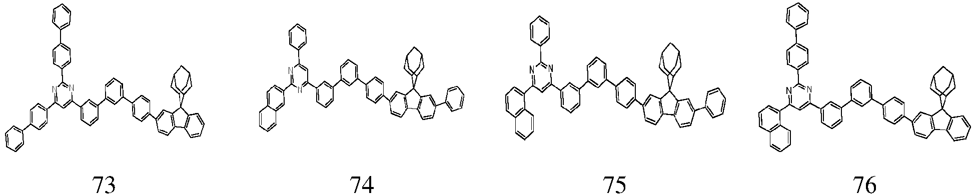

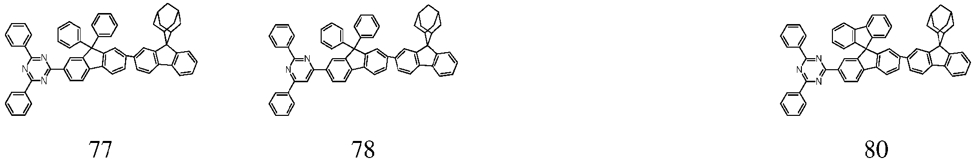

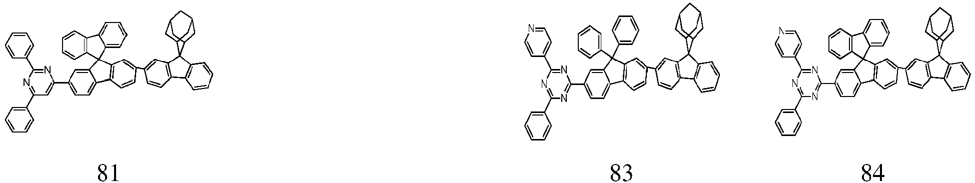

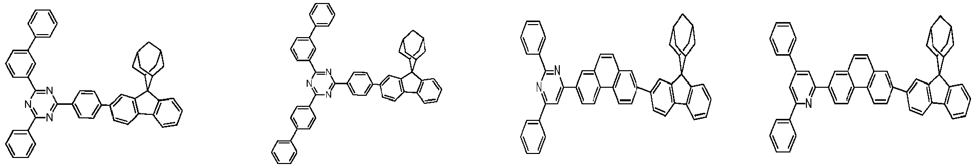

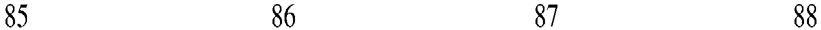

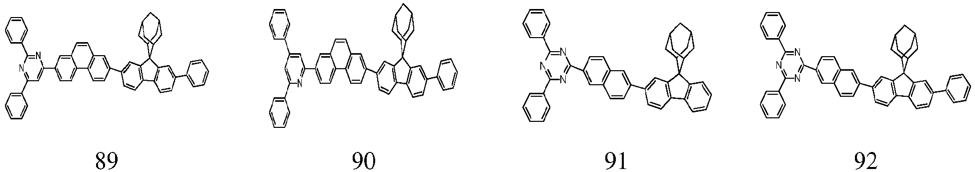

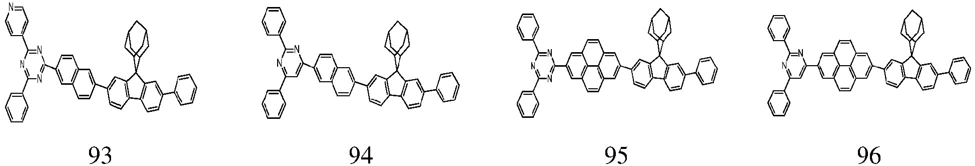

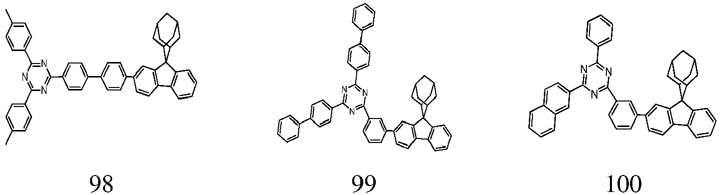

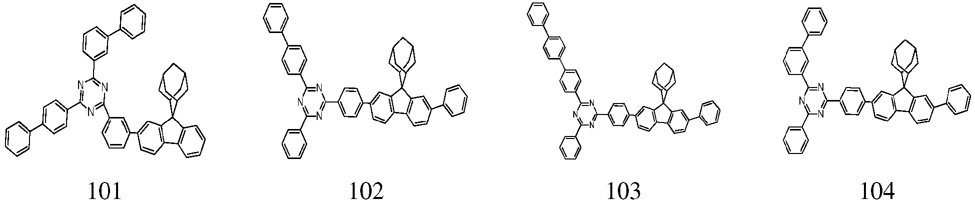

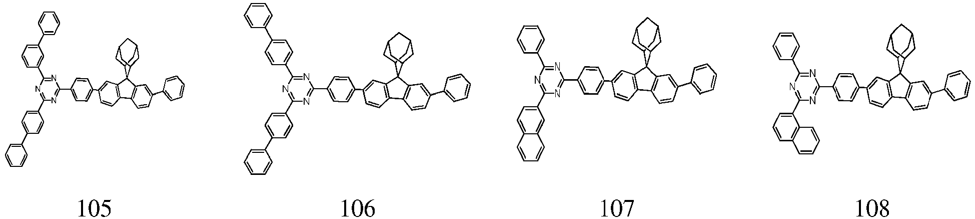

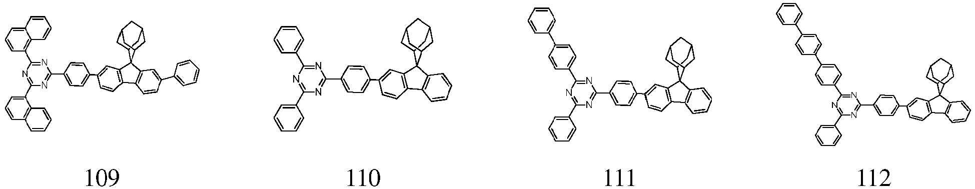

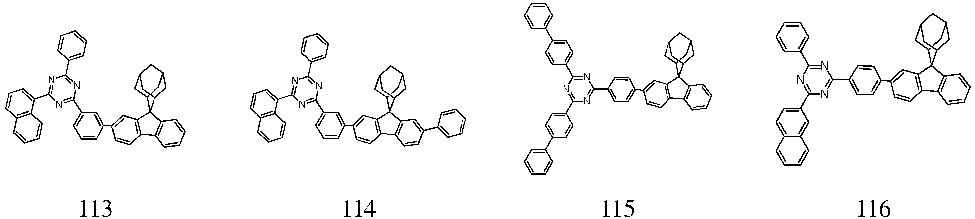

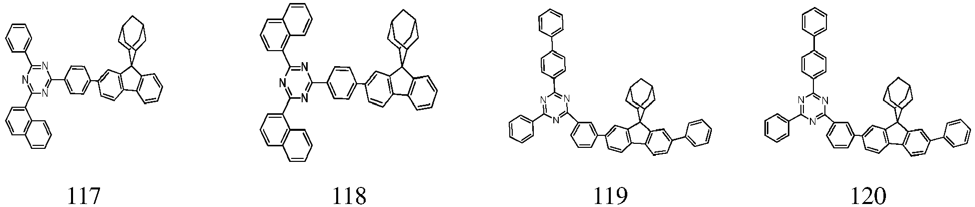

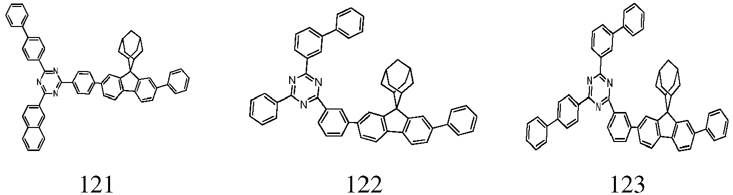

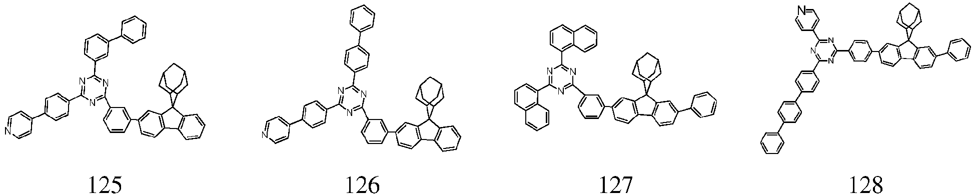

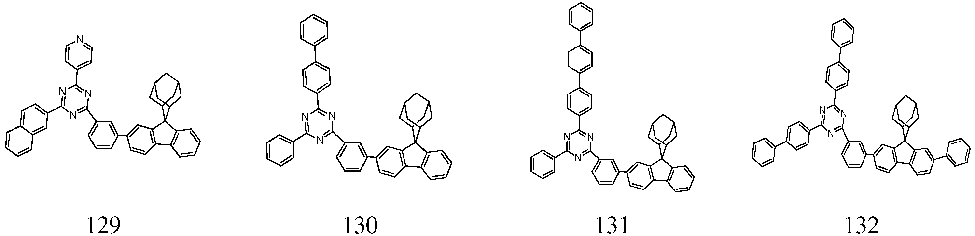

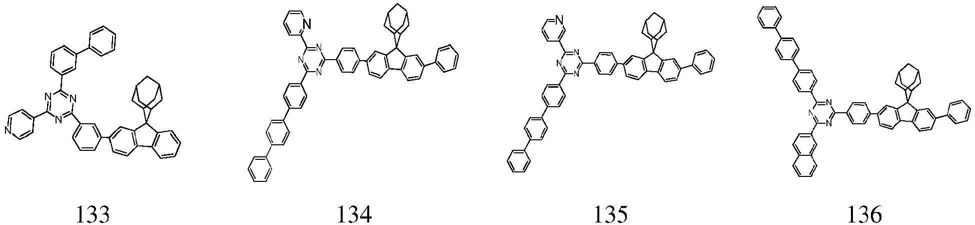

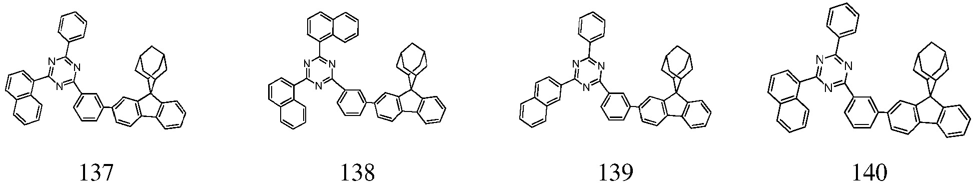

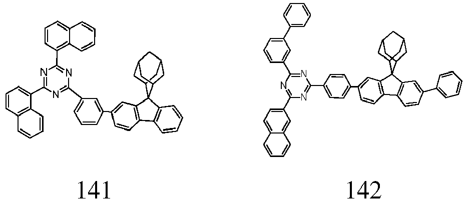

8. A photoelectric conversion device, comprising an anode, a cathode arranged opposite the anode, and a functional layer arranged between the anode and the cathode;
wherein the functional layer comprises the compound according to any one of claims 1 to 7;
optionally, the functional layer comprises an electron transport layer, and the electron transport layer comprises the compound according to any one of claims 1 to 7;
optionally, the functional layer further comprises:
a light emitting layer arranged on a side of the electron transport layer close to the anode;
a hole transport layer arranged on a side of the light emitting layer away from the cathode.

9. The photoelectric conversion device according to claim 8, wherein the photoelectric conversion device is an organic electroluminescent device or a solar cell.

10. An electronic apparatus, comprising the photoelectric conversion device according to any one of claims 8 to 9.

## Patentansprüche

1. Eine Verbindung, wobei die allgemeine Strukturformel der Verbindung in Formel 1 dargestellt ist: 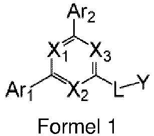 worin Y ausgewählt ist aus: 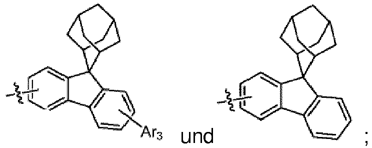
X₁ und X₂ unabhängig voneinander aus Kohlenstoff und Stickstoff ausgewählt sind; X₃ Stickstoff ist;
L aus einer Einfachbindung, substituiertem oder unsubstituiertem Arylen mit 6 bis 30 Kohlenstoffatomen, substituiertem oder unsubstituiertem Heteroarylen mit 2 bis 29 Kohlenstoffatomen, substituiertem oder unsubstituiertem Arylalkylen mit 7 bis 30 Kohlenstoffatomen und substituiertem oder unsubstituiertem Heteroarylalkylen mit 3 bis 29 Kohlenstoffatomen ausgewählt ist;
Ar₁ , Ar₂ und Ar₃ gleich oder verschieden sind und unabhängig voneinander aus substituiertem oder unsubstituiertem Arylalkyl mit 7 bis 30 Kohlenstoffatomen, substituiertem oder unsubstituiertem Heteroarylalkyl mit 2 bis 29 Kohlenstoffatomen, substituiertem oder unsubstituiertem Aryl mit 6 bis 30 Kohlenstoffatomen und substituiertem oder unsubstituiertem Heteroaryl mit 2 bis 30 Kohlenstoffatomen ausgewählt sind;
die Substituenten von Ar₁ , Ar₂, Ar₃ und L gleich oder verschieden sind und unabhängig voneinander aus Deuterium, Cyano, Nitro, Halogen, Hydroxyl, substituiertem oder unsubstituiertem Alkyl mit 1 bis 24 Kohlenstoffatomen, substituiertem oder unsubstituiertem Cycloalkyl mit 3 bis 24 Kohlenstoffatomen, substituiertem oder unsubstituiertem Alkenyl mit 2 bis 24 Kohlenstoffatomen, substituiertem oder unsubstituiertem Alkynyl mit 2 bis 24 Kohlenstoffatomen, substituiertem oder unsubstituiertem Heterocycloalkyl mit 2 bis 24 Kohlenstoffatomen, substituiertem oder unsubstituiertem Arylalkyl mit 7 bis 24 Kohlenstoffatomen, substituiertem oder unsubstituiertem Heteroarylalkyl mit 2 bis 24 Kohlenstoffatomen, substituiertem oder unsubstituiertem Aryl mit 6 bis 24 Kohlenstoffatomen, substituiertem oder unsubstituiertem Heteroaryl mit 1 bis 24 Kohlenstoffatomen, substituiertem oder unsubstituiertem Alkoxy mit 1 bis 24 Kohlenstoffatomen, substituiertem oder unsubstituiertem Alkylamino mit 1 bis 24 Kohlenstoffatomen, substituiertem oder unsubstituiertem Arylamino mit 6 bis 24 Kohlenstoffatomen, substituiertem oder unsubstituiertem Alkylthio mit 1 bis 24 Kohlenstoffatomen, substituiertem oder unsubstituiertem Arylalkylamino mit 7 bis 24 Kohlenstoffatomen, substituiertem oder unsubstituiertem Heteroarylamino mit 1 bis 24 Kohlenstoffatomen, substituiertem oder unsubstituiertem Alkylsilyl mit 1 bis 24 Kohlenstoffatomen, substituiertem oder unsubstituiertem Arylsilyl mit 6 bis 24 Kohlenstoffatomen, substituiertem oder unsubstituiertem Aryloxy mit 6 bis 24 Kohlenstoffatomen, und substituiertem oder unsubstituiertem Arylthiol mit 6 bis 24 Kohlenstoffatomen ausgewählt sind.

2. Die Verbindung nach Anspruch 1, wobei L aus Arylen mit 6 bis 25 ringbildenden Kohlenstoffatomen ausgewählt ist.

3. Die Verbindung nach Anspruch 1 oder 2, wobei L aus der Gruppe bestehend aus den folgenden Gruppen ausgewählt ist: 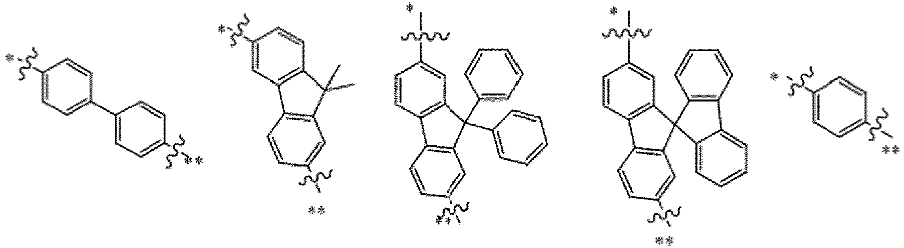 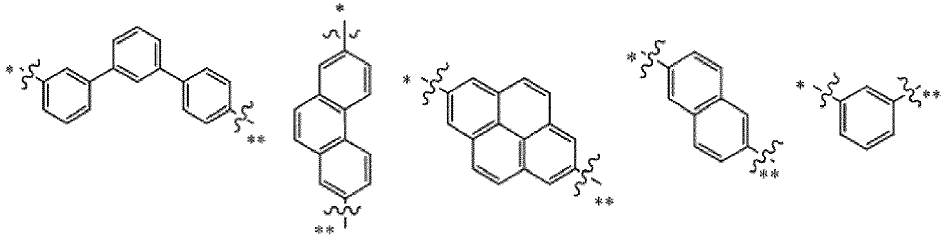 worin * ein Verbindungsglied mit der Gruppe von 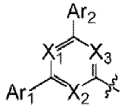 in Formel 1 darstellt; ** ein Verbindungsglied mit der Gruppe von 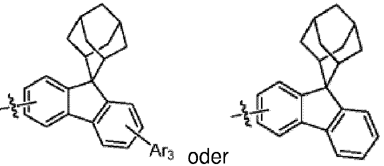 in Formel 1 darstellt.

4. Die Verbindung nach einem der Ansprüche 1 bis 3, wobei Ar₁ und Ar₂ gleich oder verschieden sind und unabhängig voneinander aus substituiertem oder unsubstituiertem Aryl mit 6 bis 30 Kohlenstoffatomen und substituiertem oder unsubstituiertem Heteroaryl mit 2 bis 29 Kohlenstoffatomen ausgewählt sind.

5. Die Verbindung nach einem der Ansprüche 1 bis 4, wobei Ar₁ und Ar₂ gleich oder verschieden sind und unabhängig voneinander aus substituiertem oder unsubstituiertem Aryl mit 6 bis 12 ringbildenden Kohlenstoffatomen und substituiertem oder unsubstituiertem Heteroaryl mit 5 ringbildenden Kohlenstoffatomen ausgewählt sind.

6. Die Verbindung nach einem der Ansprüche 1 bis 5, wobei Ar₁ und Ar₂ gleich oder verschieden sind und unabhängig voneinander aus der Gruppe der folgenden Gruppen ausgewählt sind: 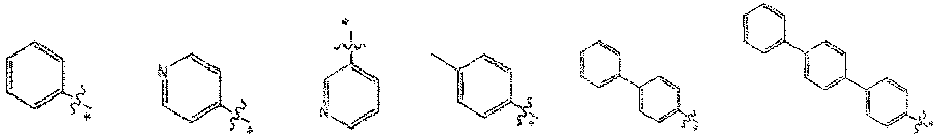 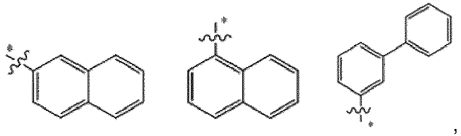 worin * ein Verbindungsglied mit der Gruppe von 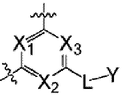 in Formel 1 darstellt.

7. Die Verbindung nach einem der Ansprüche 1 bis 6, wobei die Verbindung aus der Gruppe bestehend aus den folgenden Verbindungen ausgewählt ist:

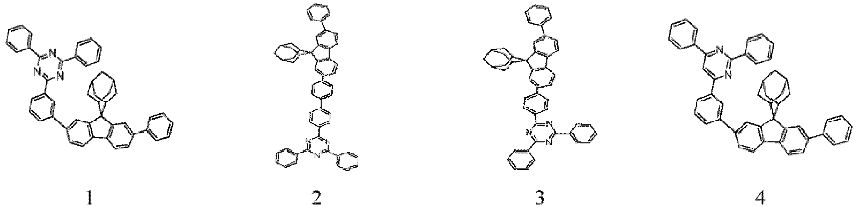

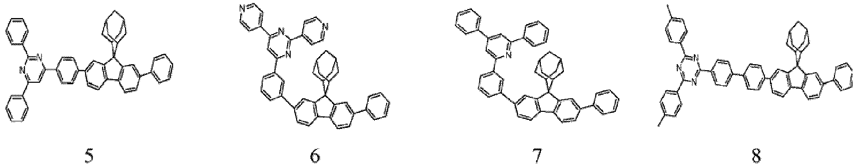

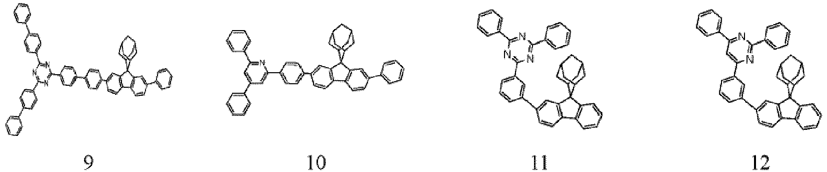

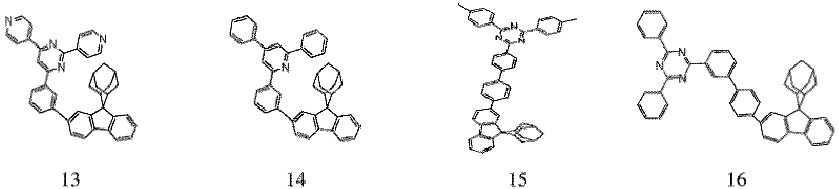

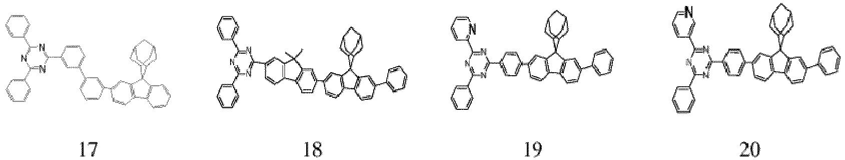

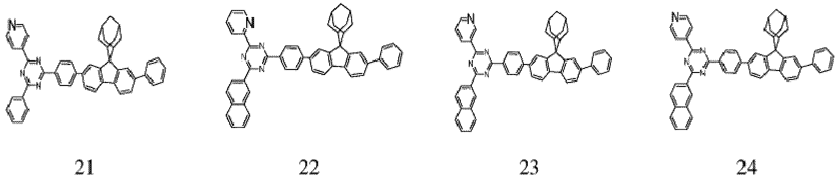

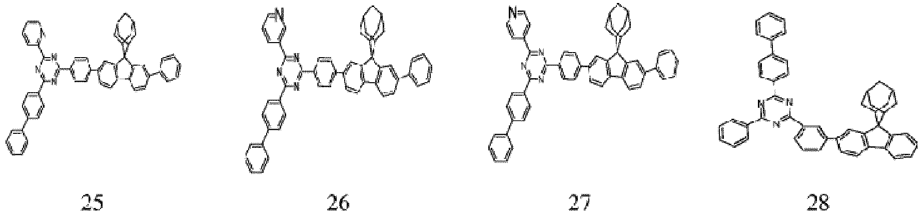

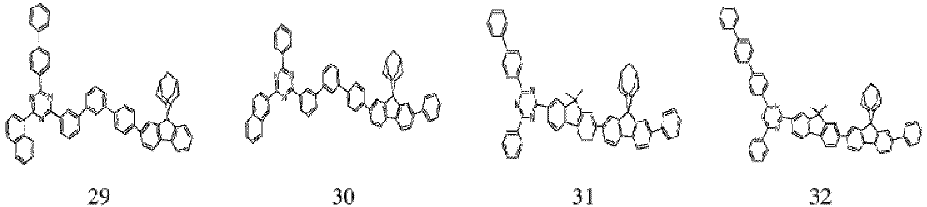

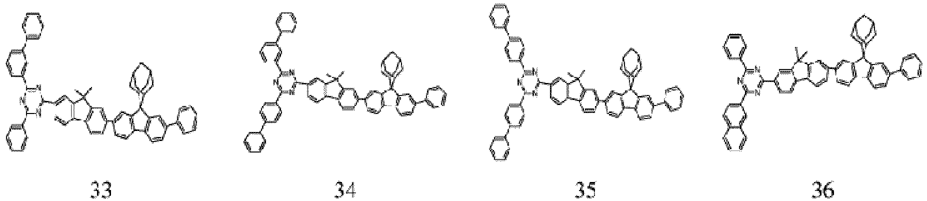

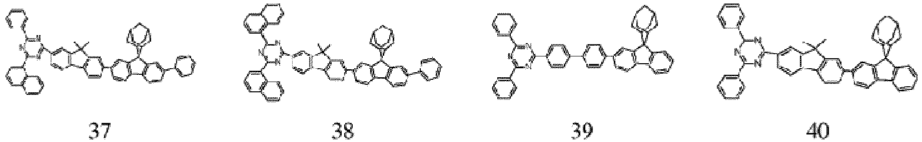

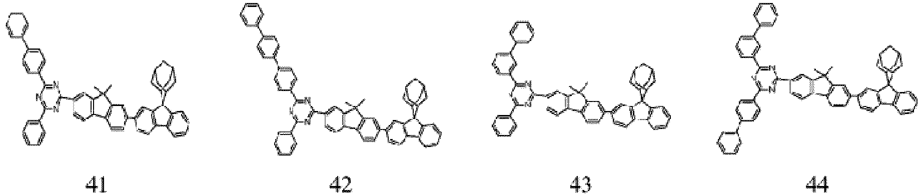

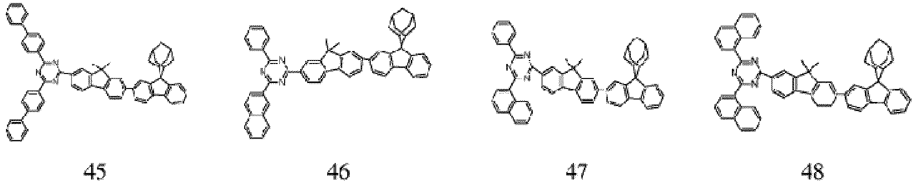

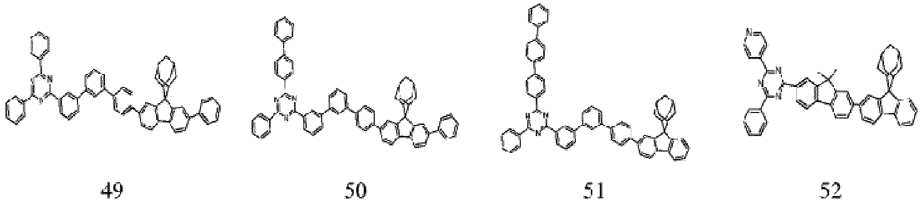

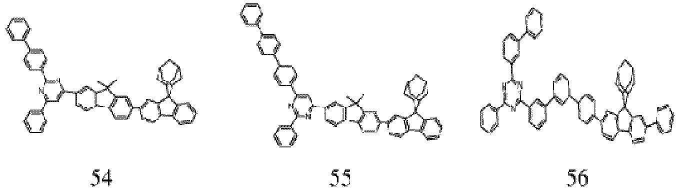

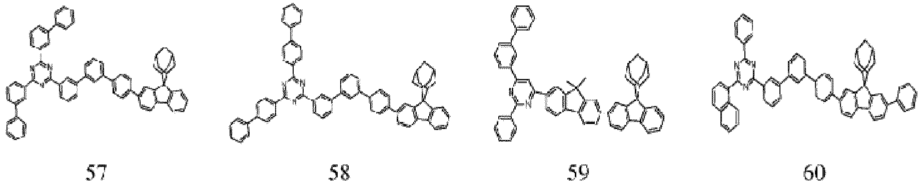

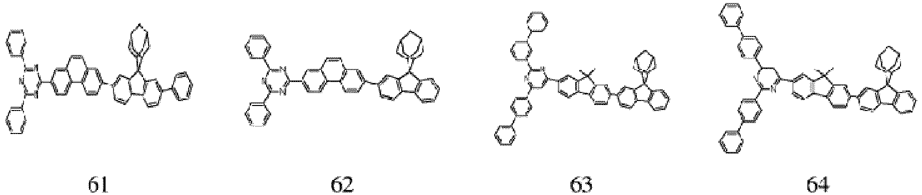

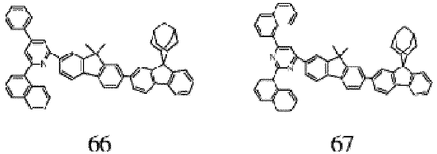

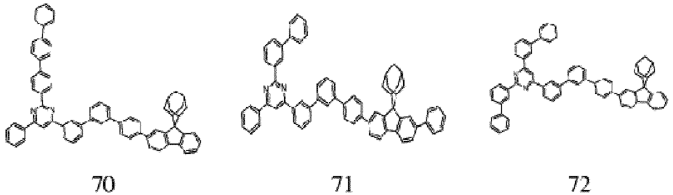

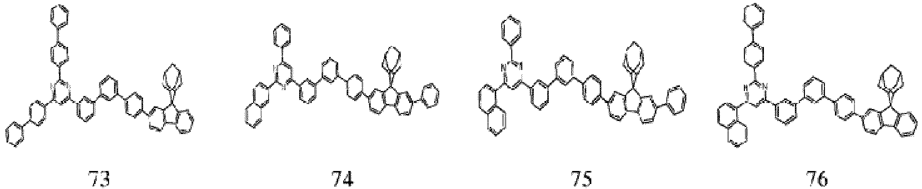

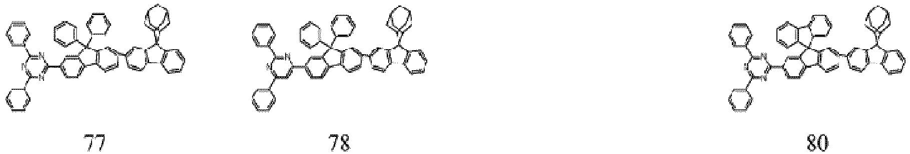

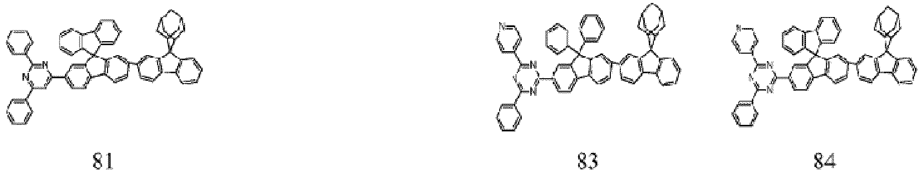

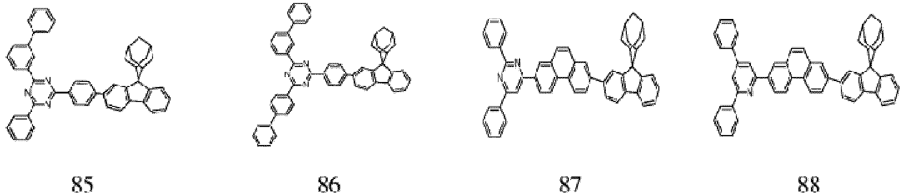

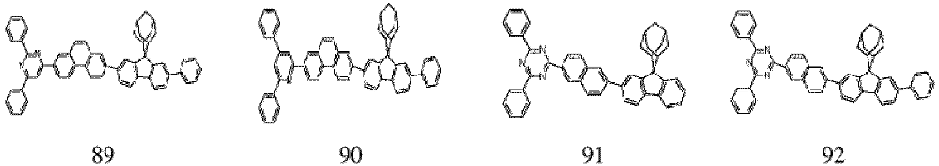

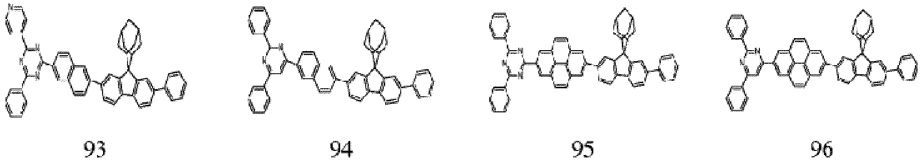

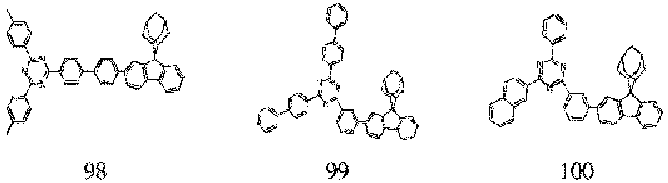

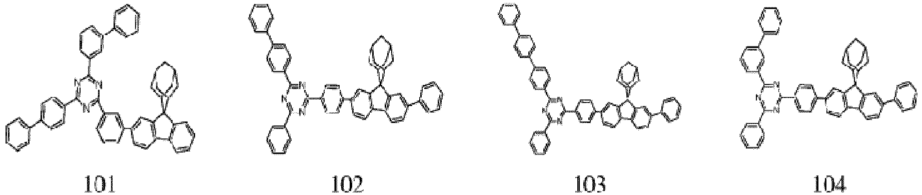

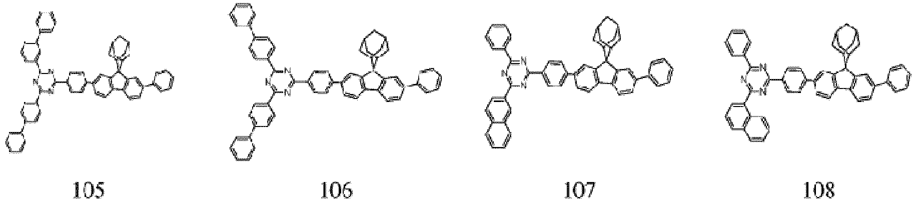

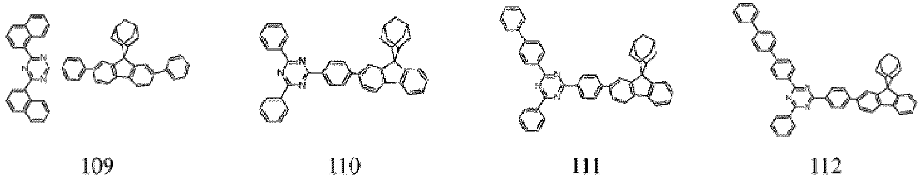

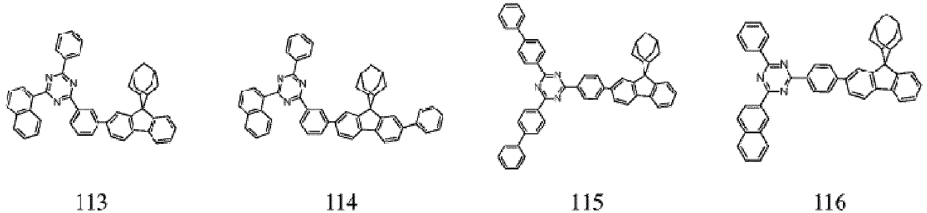

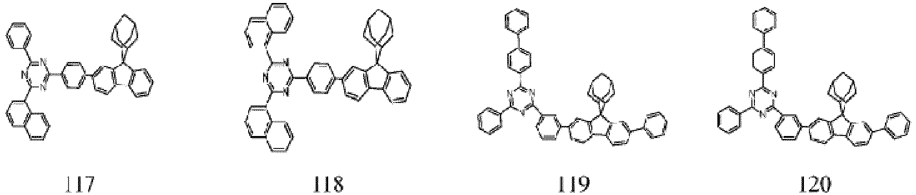

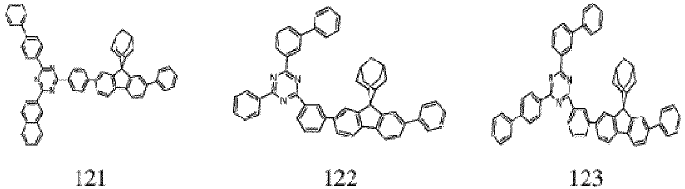

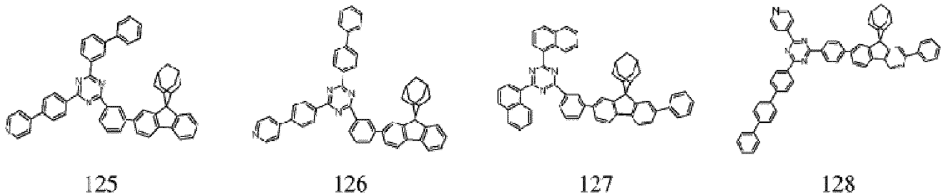

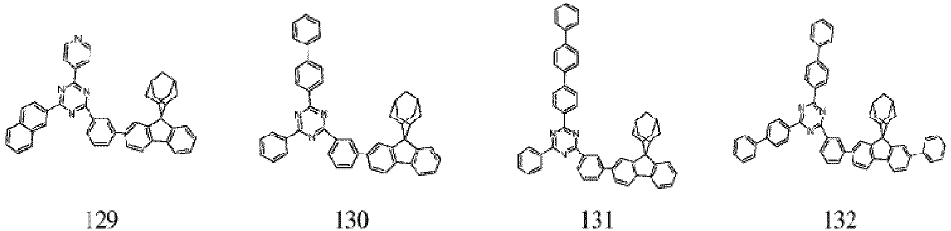

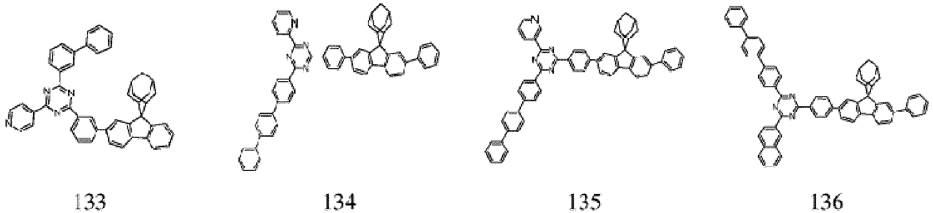

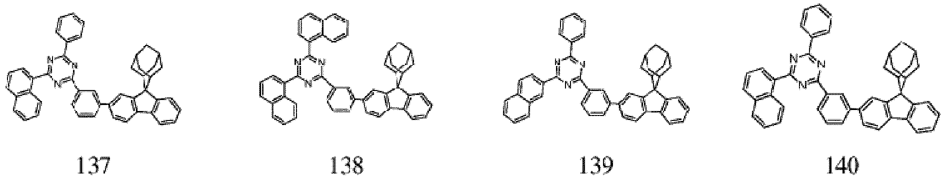

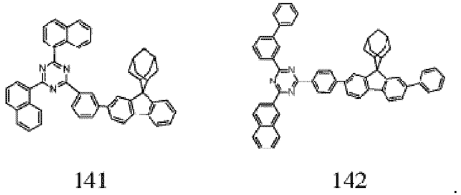

8. Eine photoelektrische Wandlungsvorrichtung umfassend eine Anode, eine gegenüber der Anode angeordnete Kathode und eine zwischen der Anode und der Kathode angeordnete Funktionsschicht;
wobei die Funktionsschicht die Verbindung nach einem der Ansprüche 1 bis 7 umfasst; die Funktionsschicht optional eine Elektronentransportschicht umfasst und die Elektronentransportschicht die Verbindung nach einem der Ansprüche 1 bis 7 umfasst;
die Funktionsschicht ferner optional folgendes umfasst:
eine lichtemittierende Schicht, die auf einer Seite der Elektronentransportschicht in der Nähe der Anode angeordnet ist;
eine Lochtransportschicht, die auf einer von der Kathode abgewandten Seite der lichtemittierenden Schicht angeordnet ist.

9. Die photoelektrische Wandlungsvorrichtung nach Anspruch 8, wobei die photoelektrische Wandlungsvorrichtung eine organische Elektrolumineszenzvorrichtung oder eine Solarzelle ist.

10. Ein elektronisches Gerät, das die photoelektrische Wandlungsvorrichtung nach einem der Ansprüche 8 bis 9 umfasst.

## Revendications

1. Composé, dans lequel la formule structurelle générale du composé est représentée par la formule 1 : 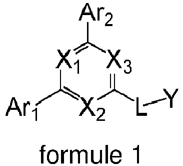 dans lequel Y est choisi parmi : 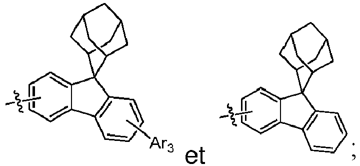
X₁ et X₂ sont indépendamment choisis parmi le carbone et l'azote ; X₃ est l'azote ;
L est choisi parmi une liaison simple, un arylène substitué ou non substitué de 6 à 30 atomes de carbone, un hétéroarylène substitué ou non substitué de 2 à 29 atomes de carbone, un arylalkylène substitué ou non substitué de 7 à 30 atomes de carbone, et un hétéroarylalkylène substitué ou non substitué de 3 à 29 atomes de carbone ;
An, Ar₂ et Ar₃ sont identiques ou différents, et sont indépendamment choisis parmi un arylalkyle substitué ou non substitué de 7 à 30 atomes de carbone, un hétéroarylalkyle substitué ou non substitué de 2 à 29 atomes de carbone, un aryle substitué ou non substitué de 6 à 30 atomes de carbone, et un hétéroaryle substitué ou non substitué de 2 à 30 atomes de carbone ;
les substituants de An, Ar₂, Ar₃ et L sont identiques ou différents, et sont indépendamment choisis parmi le deutérium, cyano, nitro, un halogène, hydroxyle, un alkyle substitué ou non substitué de 1 à 24 atomes de carbone, un cycloalkyle substitué ou non substitué de 3 à 24 atomes de carbone, un alcényle substitué ou non substitué de 2 à 24 atomes de carbone, un alcynyle substitué ou non substitué de 2 à 24 atomes de carbone, un hétérocycloalkyle substitué ou non substitué de 2 à 24 atomes de carbone, un arylalkyle substitué ou non substitué de 7 à 24 atomes de carbone, un hétéroarylalkyle substitué ou non substitué de 2 à 24 atomes de carbone, un aryle substitué ou non substitué de 6 à 24 atomes de carbone, un hétéroaryle substitué ou non substitué de 1 à 24 atomes de carbone, un alcoxy substitué ou non substitué de 1 à 24 atomes de carbone, un alkylamino substitué ou non substitué de 1 à 24 atomes de carbone, un arylamino substitué ou non substitué de 6 à 24 atomes de carbone, un alkylthio substitué ou non substitué de 1 à 24 atomes de carbone, un arylalkylamino substitué ou non substitué de 7 à 24 atomes de carbone, un hétéroarylamino substitué ou non substitué de 1 à 24 atomes de carbone, un alkylsilyle substitué ou non substitué de 1 à 24 atomes de carbone, un arylsilyle substitué ou non substitué de 6 à 24 atomes de carbone, un aryloxy substitué ou non substitué de 6 à 24 atomes de carbone, et un arylthio substitué ou non substitué de 6 à 24 atomes de carbone.

2. Composé selon la revendication 1, dans lequel L est choisi parmi un arylène de 6 à 25 atomes de carbone formant un cycle.

3. Composé selon la revendication 1 ou 2, dans lequel L est choisi dans l'ensemble constitué par les groupes suivants : 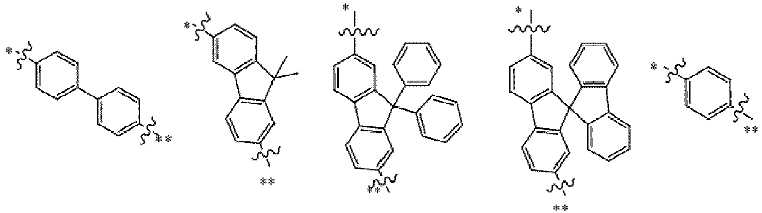 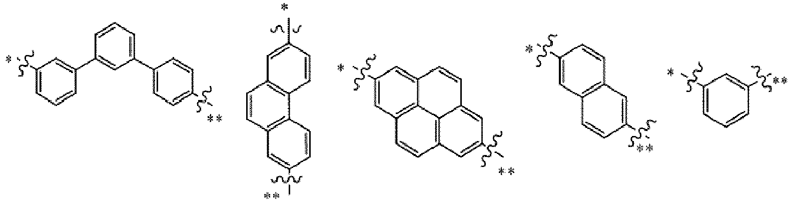 dans lequel * représente une liaison avec le groupe la formule 1 ; 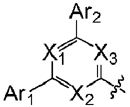 dans ** représente une liaison avec le groupe 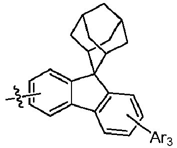 ou 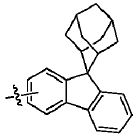 dans la formule 1.

4. Composé selon l'une quelconque des revendications 1 à 3, dans lequel Ar₁ et Ar₂ sont identiques ou différents et sont indépendamment choisis parmi un aryle substitué ou non substitué de 6 à 30 atomes de carbone, et un hétéroaryle substitué ou non substitué de 2 à 29 atomes de carbone.

5. Composé selon l'une quelconque des revendications 1 à 4, dans lequel Ar₁ et Ar₂ sont identiques ou différents, et sont indépendamment choisis parmi un aryle substitué ou non substitué de 6 à 12 atomes de carbone formant un cycle, et un hétéroaryle substitué ou non substitué de 5 atomes de carbone formant un cycle.

6. Composé selon l'une quelconque des revendications 1 à 5, dans lequel Ar₁ et Ar₂ sont identiques ou différents, et sont indépendamment choisis dans l'ensemble formé par les groupes suivants : 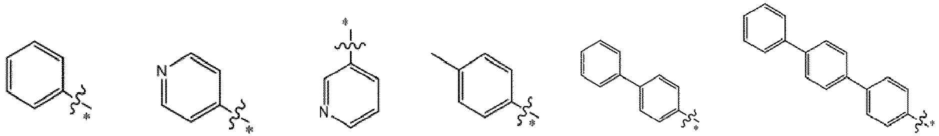 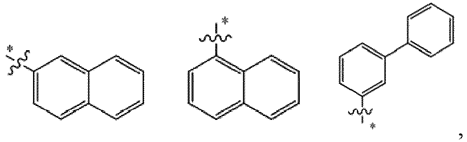 dans lequel * représente une liaison avec le groupe 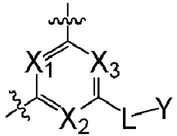 dans la formule 1.

7. Composé selon l'une quelconque des revendications 1 à 6, lequel composé est choisi dans l'ensemble constitué par les composés suivants :

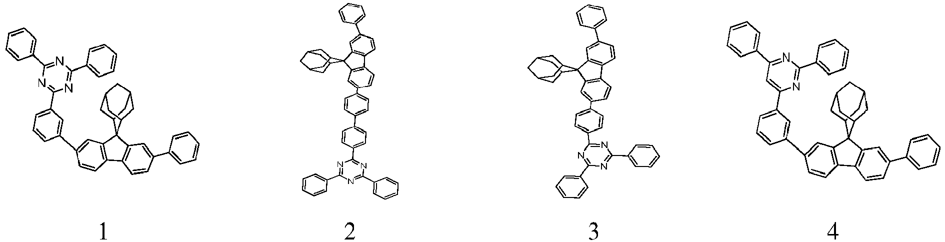

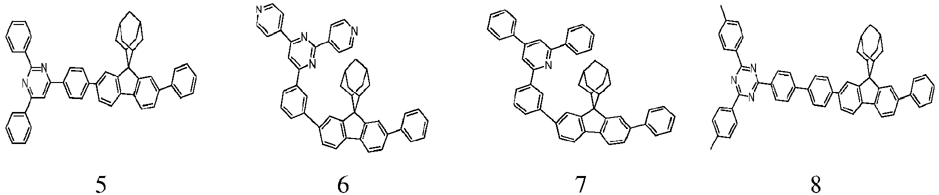

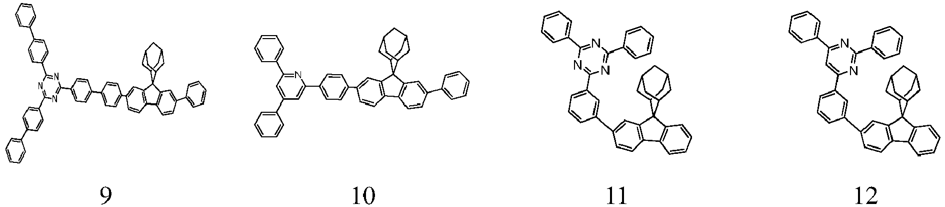

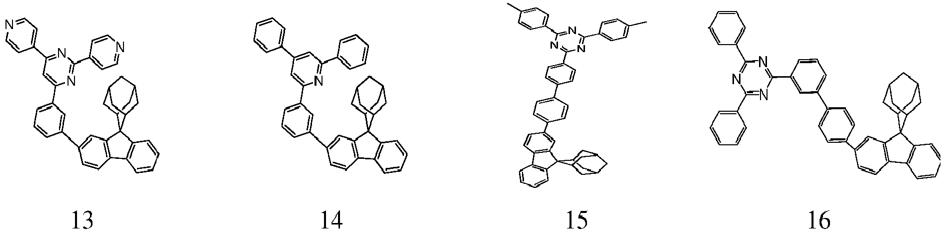

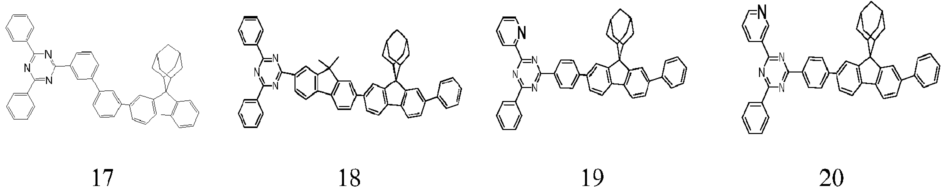

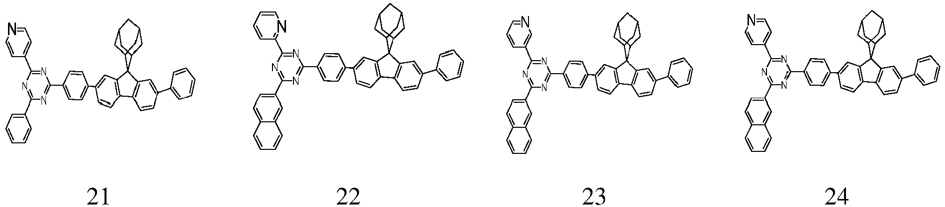

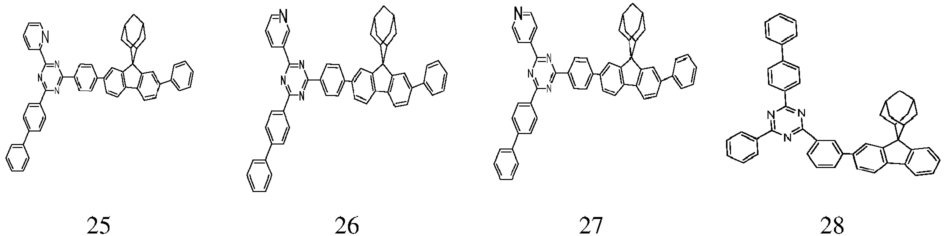

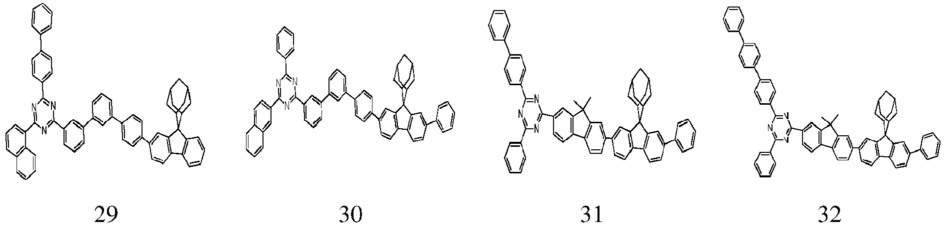

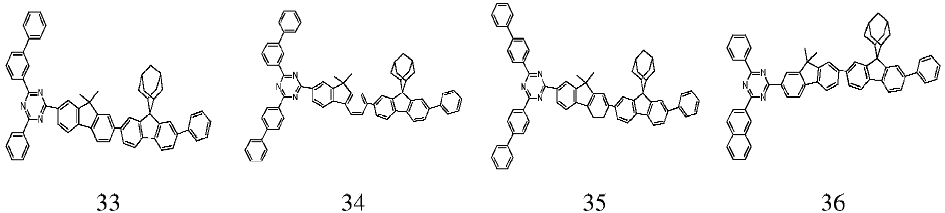

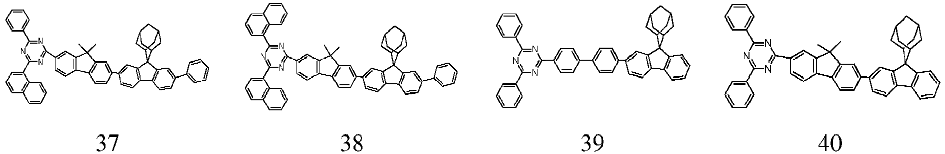

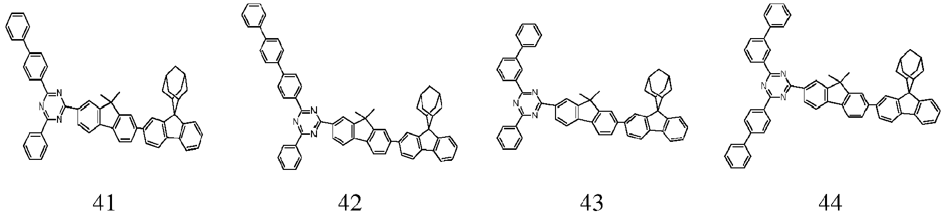

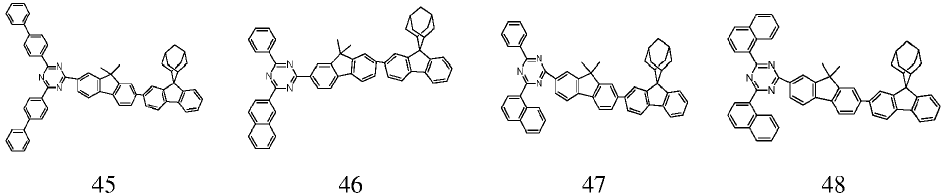

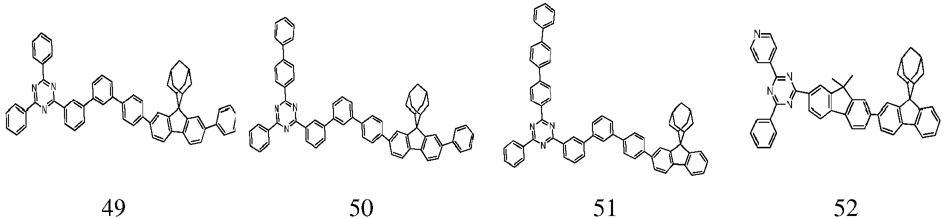

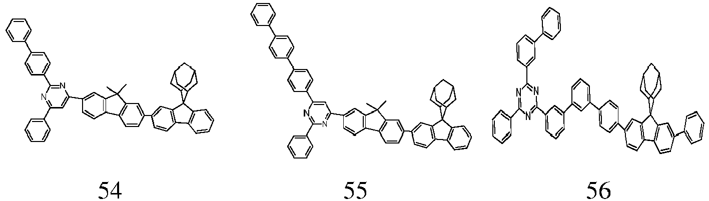

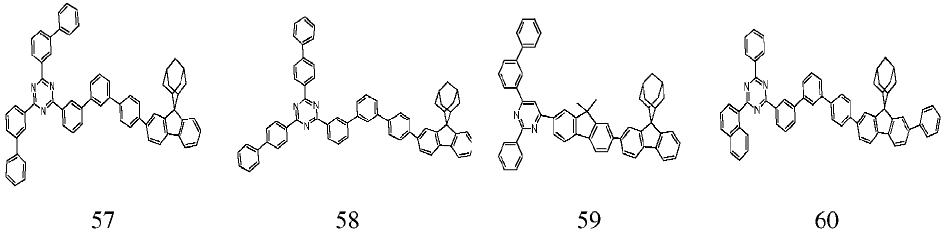

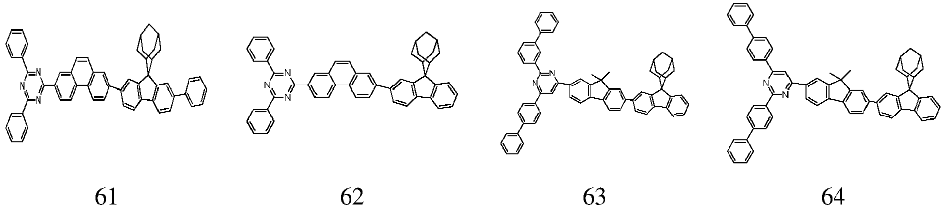

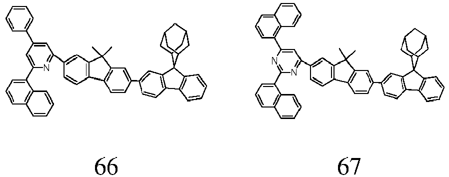

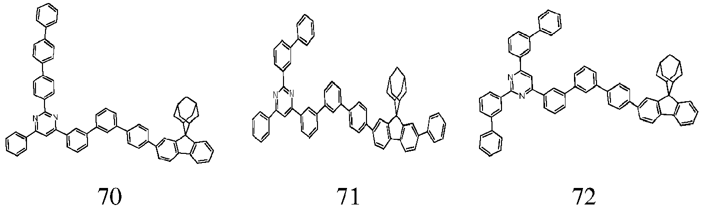

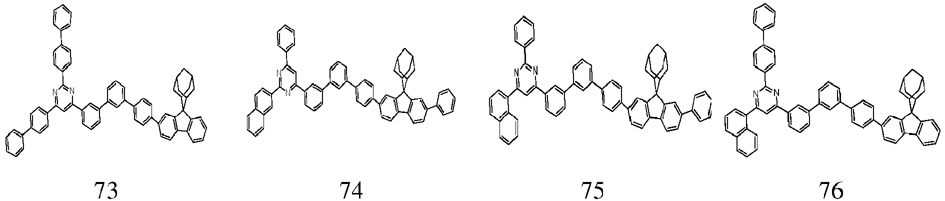

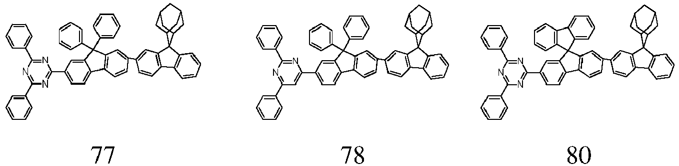

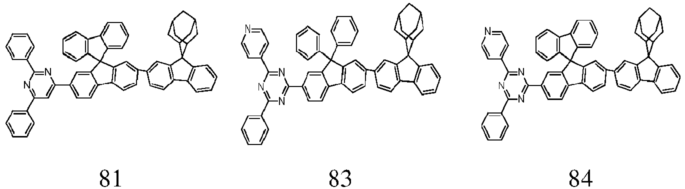

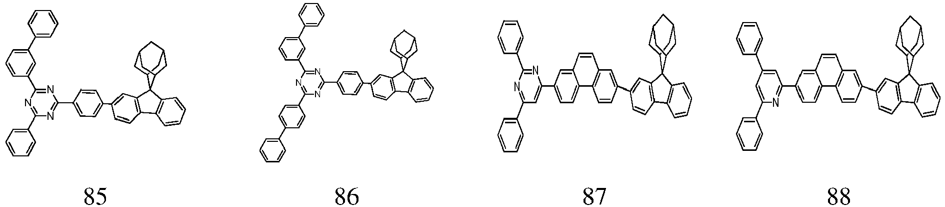

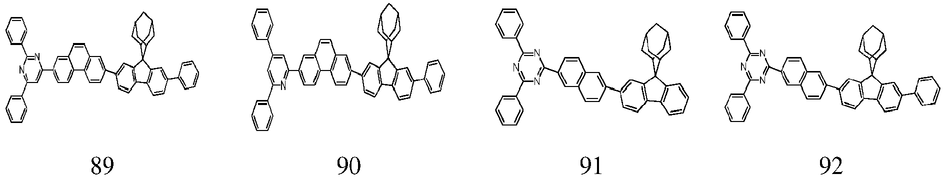

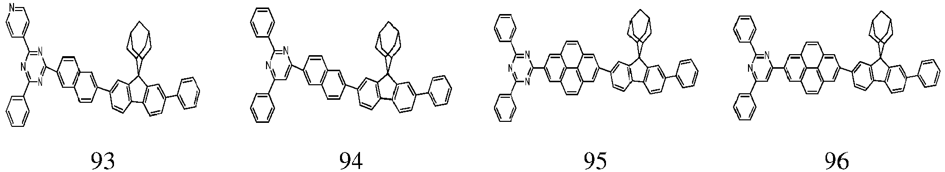

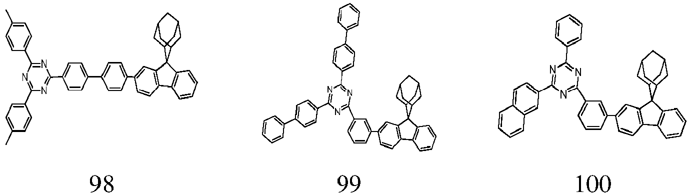

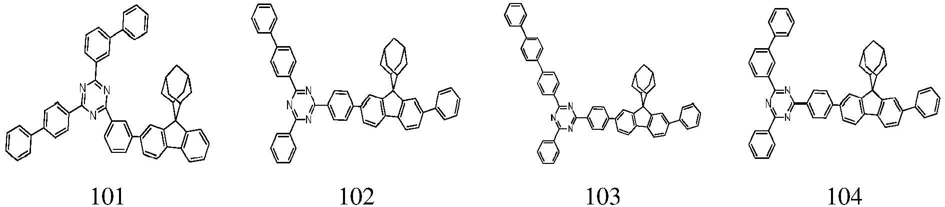

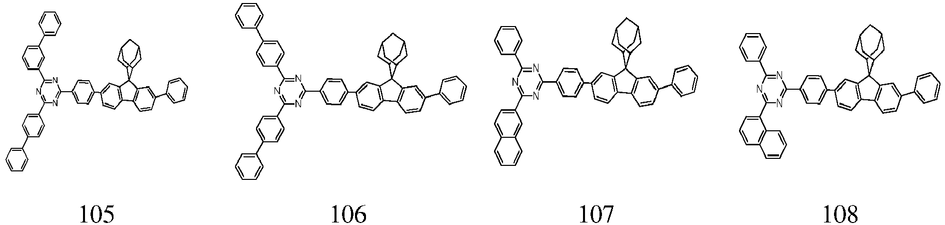

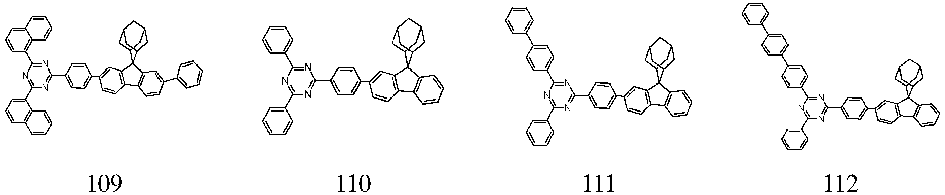

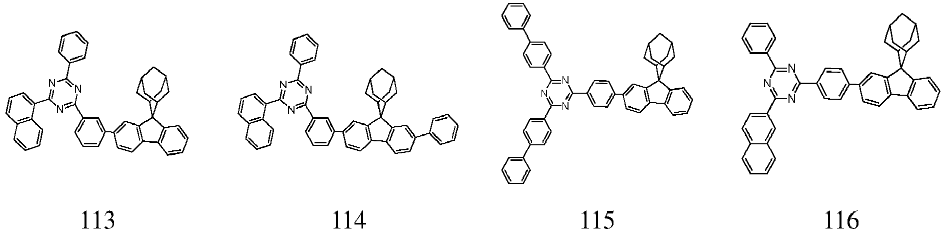

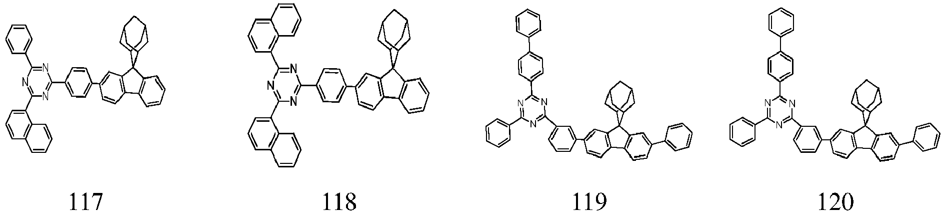

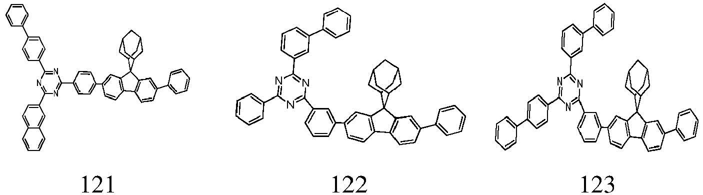

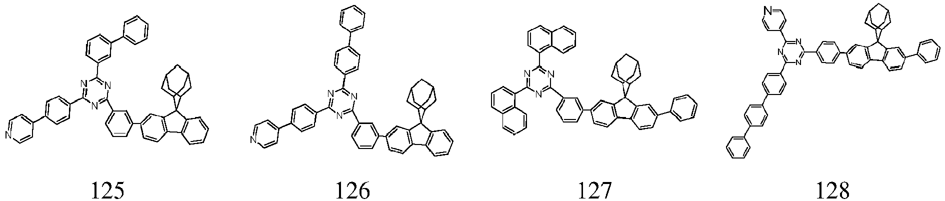

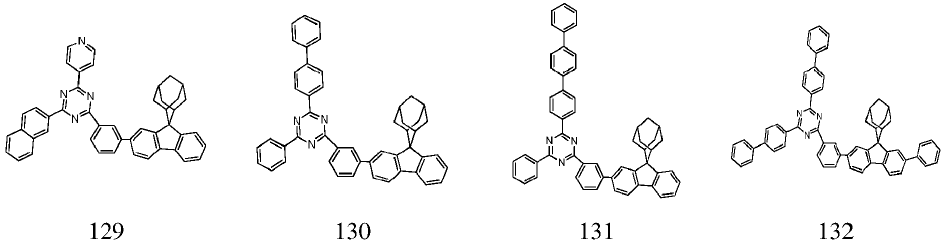

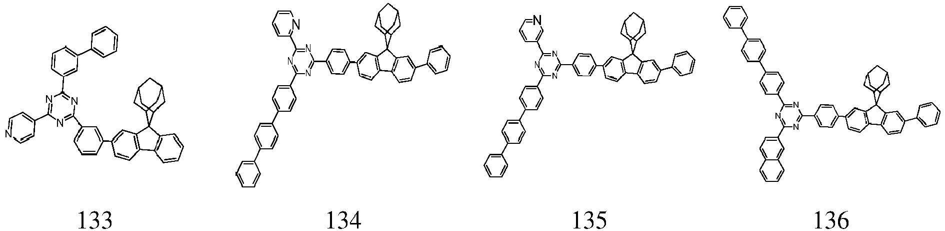

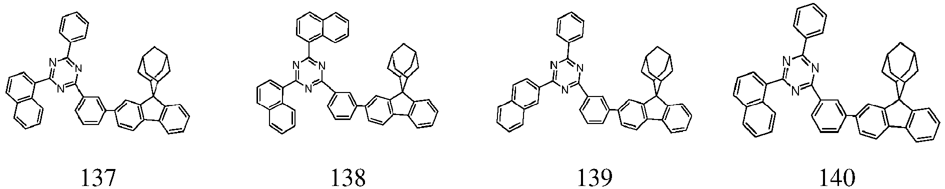

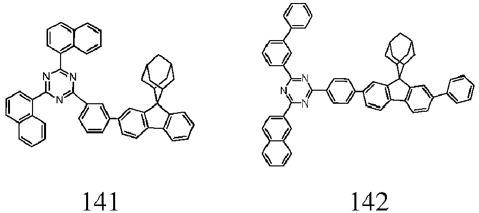

8. Dispositif de conversion photoélectrique, comprenant une anode, une cathode disposée à l'opposé de l'anode, et une couche fonctionnelle disposée entre l'anode et la cathode ;
dans lequel la couche fonctionnelle comprend le composé selon l'une quelconque des revendications 1 à 7 ;
éventuellement, la couche fonctionnelle comprend une couche de transport d'électrons, et la couche de transport d'électrons comprend le composé selon l'une quelconque des revendications 1 à 7 ;
éventuellement, la couche fonctionnelle comprend en outre :
une couche luminescente disposée sur un côté de la couche de transport d'électrons proche de l'anode ;
une couche de transport de trous disposée sur un côté de la couche de transport d'électrons éloigné de la cathode.

9. Dispositif de conversion photoélectrique selon la revendication 8, lequel dispositif de conversion photoélectrique est un dispositif électroluminescent organique ou une cellule solaire.

10. Appareil électronique comprenant le dispositif de conversion photoélectrique selon l'une quelconque des revendications 8 et 9.
